# EUROPEAN PATENT SPECIFICATION

(11) **EP 1 056 486 B1**
(45) Date of publication and mention of the grant of the patent: **11.10.2006**
(21) Application number: 98901283.6
(22) Date of filing: 29.01.1998
(51) Int. Cl.: A61L 27/00, C01B 33/24, C01B 35/08

(54) **A SYNTHETIC BIOMATERIAL COMPOUND**
SYNTHETISCHES BIOMATERIAL
COMPOSE DE BIOMATERIAU SYNTHETIQUE

(43) Date of publication of application: 06.12.2000
(73) Proprietor: MILLENIUM BIOLOGIX INC., Kingston, Ontario K7M 7G3 (CA)
(72) Inventor: PUGH, Sydney, M., Glenburnie, Ontario K0H 1S0 (CA); SMITH, Timothy, J., N., Kingston, Ontario K7M 1M1 (CA); SAYER, Michael, Kingston, Ontario K7M 1E4 (CA); LANGSTAFF, Sarah, Dorthea, Kingston, Ontario K7L 3W6 (CA)
(74) Representative: Curtis, Philip Anthony
(86) International application number: PCT/CA1998/000046
(87) International publication number: WO 1999/038542

(56) References cited:
- WO-A-91/17965
- WO-A-95/32008
- FR-A- 2 664 501
- GB-A- 2 316 940
- US-A- 4 503 157
- RUYS A J: "SILICON-DOPED HYDROXYAPATITE" JOURNAL OF THE AUSTRALIAN CERAMIC SOCIETY, vol. 29, no. 1/02, 1993, pages 71-80, XP002048629
- BOYER L ET AL: "SYNTHESIS OF PHOSPHATE - SILICATE APATITES AT ATMOSPHERIC PRESSURE" SOLID STATE IONICS, vol. 95, no. 1/02, 30 November 1995, pages 121-129, XP002048976

## Description

### Field of the Invention

The present invention is directed to a synthetic biomaterial compound based on stabilized calcium phosphates and more particularly to the molecular, structural and physical characterization of this compound, herein called Skelite™.

### Background of the Invention

It has long been the goal of biomaterials research in the field of orthopedics to develop synthetic structures exhibiting comprehensive bioactivity. Bioactive synthetic substrates capable of incorporation into the natural process of bone remodeling are of interest in applications which include *in vitro* bone cell assays [1], *in vivo* resorbable bone cements [2, 3], implantable coatings which enhance the bonding of natural bone to the implant [4], various forms of implantable prostheses and bone repair agents [5, 6], and *ex vivo* tissue engineering [7]. The prime objective for such materials *in vivo* is to combine the stimulation of osteogenic activity in associated bone tissues for optimum healing, with the capability to be progressively resorbed by osteoclasts during normal continuous remodeling [8]. *In vitro,* related functions are to provide standardized laboratory test substrates on which osteoclast resorptive function or osteoblast production of mineralized bone matrix can be assessed and quantified [1]. Such substrates must be stable and insoluble in the biological environment until acted upon by osteoclasts, the specific bone mineral resorbing cells.

While calcium hydroxyapatite (Ca₅(OH)(PO₄)₃ or HA) is the primary inorganic component of natural bone [9], trace elements are also present [10]. Calcium hydroxyapatite is but one of a number of calcium-phosphorous (Ca-P) compounds which are biocompatible. Others include octacalcium phosphate [11] and both phases of tricalcium phosphate (Ca₃(PO₄)₂ or α-TCP /β-TCP) [12]. Compounds, particularly HA, may show differing degrees of stoichiometry with the Ca/P ratio ranging from 1.55 to 2.2 [13]. Such materials can be artificially created by conventional high temperature ceramic processing [14] or by low temperature aqueous chemistry [15, 16]. Most of such artificial materials show good biocompatibility in that bone cells tolerate their presence with few deleterious effects, and indeed enhanced bone deposition may occur [17,18]. Currently, the most recognized medical application of calcium phosphates is the coating of implantable prosthetic devices and components by thermal or plasma spray to render the surface osteoconductive. It has been noted that Ca-P ceramics which are stable in biological environments are often a mixture of individual compounds [19]. However, despite the osteogenic potential of these artificial materials, none actively participate in the full process of natural bone remodeling.

In the applicant's published international PCT application WO94/26972, cell-mediated resorption was shown to occur on a calcium phosphate-based thin film formed by the high temperature processing of a calcium phosphate colloidal suspension on quartz substrates. When used *in vitro,* these ceramic films exhibited multiple discrete resorption events (lacunae) across their surface as a result of osteoclast activity, with no evidence of dissolution arising from the culture medium. The regular margins of these lacunae correspond closely to the size and shape of the ruffled borders normally produced by osteoclasts as the means by which they maintain the localized low pH required to naturally resorb bone mineral *in vivo.* Enhanced deposition of mineralized bone matrix also occurs on these ceramics in the presence of osteoblasts.

It was later revealed in the Applicant's published international PCT application WO 97/09286, that these thin film ceramics exhibited two general characteristics: (1) the presence of a mixture of Ca-P containing phases comprising approximately 33% HA and approximately 67% of a silicon stabilized calcium phosphate and (2) a unique morphology. Importantly, it was noted that the thermal processing of the Ca-P. colloid at 1000°C resulted in an HA powder, while the same colloidal suspension processed on quartz had a mixed HA and silicon stabilized calcium phosphate phase composition. Energy dispersive X-ray analysis of the film demonstrated the presence of Si in the coating while cross-sectional transmission electron microscopy indicated a microporous physical structure.

In view of the clinical importance of developing a synthetic bone graft that is both osteogenic and can participate in the body's natural cell-based remodeling process, it was important to focus on the role of introduced additives such as silicon in the formation of a calcium phosphate-based biomaterial compound capable of being assimilated and remodeled into natural bone with the aid of the activity of osteoclasts and osteoblasts. Since the compound could only be characterized by the preparation method, it was crucial to be able to chemically characterize the compound beyond its microporous physical structure. In particular, it was important to characterize the specific molecular and chemical structure of the stabilized compound in order to be able to understand why the new compound worked so well in biological conditions affecting the skeleton. The molecular and chemical characterization of the compound could also provide for the development of further uses of the compound in the treatment of several different types of bone-related clinical conditions. In addition, this would also allow further chemical alteration of the compound in order that it could be designed for use in specific *in vivo, in vitro* and *ex vivo* applications.

The Applicants previous published work in WO 94/26872 and WO 97/09286 had appointed to the transformation of HA into a stabilized α-TCP phase. Surprisingly, during the difficult course of explicit characterization of the compound from a molecular standpoint, it was found that the resultant stabilized compound was in fact an entirely new compound herein described and termed Skelite™.

### Summary on the Invention

Stabilized calcium phosphate-based thin films and bulk ceramics have been created and have only now been specifically characterized with respect to their physical and chemical structure. The biomaterial compound is made by the high temperature processing of a fine precipitate, formed from a colloidal suspension and stabilized using an additive with an appropriate sized ionic radius that enables substitution into the Ca-P lattice. The compound typically co-exists with calcium hydroxyapatite and is itself a novel stabilised calcium phosphate compound using a microporous morphology based on inter-connected particles of about 0.2-1.0µm in diameter. The compound is essentially insoluble in biological media but is resorbable when acted upon by osteoblasts. It also promotes organic bone matrix deposition by osteblasts and can be assimilated into natural bone during the natural course of bone remodeling through the activity of osteoclasts and osteoblasts. The compound has been extensively analysed using X-ray diffraction, infrared spectroscopy, nuclear magnetic resonance spectroscopy, and light scattering particle analysis. Results now indicate that the characteristic features of the compound arise during sintering through substitution reactions where a stabilizing element such as silicon enters the calcium phosphate lattice under conditions of high chemical reactivity. The crystallographic features are linked through the glaserite form of the apatite structure.

According to an aspect of the present invention a biomaterial tricalcium phosphate compound is provided comprising calcium, oxygen and phosphorous, wherein at least one of the element is substituted with an element having an ionic radius of approximately 0.1 to 0.4Å.

According to another aspect of the present invention is a biomaterial compound having the formula (Ca)ᵢ[(P_{1-x-y-z}BₓC_{y}D_{z})Oⱼ]₂; wherein B, C and D are selected from those elements having an ionic radius of approximately 0.1 to 0.4Å; x is greater than or equal to zero but less than 1; y is greater than or equal to zero but less than 1; z is greater than or equal to zero but less than 1; x+y+z is greater than zero but less than 1; i is greater than or equal to 2 but less than or equal to 4; and j equals 4-δ, where δ is greater than or equal to zero but less than or equal to 1.

Specific compounds of the present invention include but are not limited to Ca₃(P_{0.1750}Si_{0.25}O_{3.875})₂ and Ca₃(P_{0.9375}Si_{0.0625}O_{3.96875})₂.

The knowledge of the specific molecular and chemical properties of the compound of the present invention allows for the development of several uses of the compound in various bone-related clinical conditions. Such applications may include orthopedic, maxillon-facial and dental applications where the compound can be fabricated to exist as a fine or coarse powder, pellets, three-dimensional shaped pieces, macroporous structures, thin films and coatings.

The biomaterial compound of the present invention may be used to substitute natural bone at sites of skeletal surgery in human and animal hosts. This method comprises the steps of implanting the biomaterial compound at the site of skeletal surgery wherein such implantation promotes the formation of new bone tissue at the interfaces between the biomaterial compound and the host, the progressive removal of the biomaterial compound primarily through osteoclast activity, and the replacement of that portion of the biomaterial compound removed by further formation of new bone tissue by osteoblast activity, such progressive removal and replacement being inherent in the natural bone remodeling process.

The biomaterial compound of the present invention may be used to repair large segmental skeletal gaps and non-union fractures arising from trauma or surgery in human and animal hosts. This method comprises the steps of implanting the biomaterial compound at the site of the segmental skeletal gap or non-union fracture wherein such implantation promotes the formation new bone tissue at the interfaces between the biomaterial compound and the host, the progressive removal of the biomaterial compound primarily through osteoclasts activity, and the replacement of that portion of the biomaterial compound removed by further formation of new bone tissue by osteoblast activity, such progressive removal and replacement being inherent in the natural bone remodelling process.

The biomaterial compound of the present invention may be used to aid the attachment of implantable prosthesis to skeletal sites and for maintaining the long term stability of the prosthesis in human and animal hosts. This method comprises the steps of coating selected regions of an implantable prosthesis with the biomaterial compound, implanting the coated prosthesis into a skeletal site wherein such implantation promotes the formation of new bone tissue at the interfaces between the biomaterial compound and the host, the generation of a secure interfacial bond between the host bone and the coating, the subsequent progressive removal of the coating primarily through osteoclast activity such that the coating is diminished, and the replacement of that portion of the biomaterial compound removed by further formation of new bone tissue to generate a secure interfacial bond directly between the host bone and the phosthesis.

The biomaterial compound of the present invention may be used to provide tissue-engineering scaffolds for bone replacement in human or animal hosts. This method comprises the steps of forming the biomaterial compound as a macroporous structure comprising an open cell construction with interconnected voids, combining mature and/or precursor bone cells with the macroporous structure, and allowing the cells to infiltrate the structure in order to develop new mineralised matrix throughtout the structure.

The knowledge of the structure of the novel compound of the present invention also allows for the use of the compound as a carrier for various pharmaceutical agents including but not restricted to bone growth factor and other agents affecting bone growth and remodelling.

The biomaterial compound of the present invention may be used to deliver pharmaceutical agents to the site of skeletal surgery in human or animal hosts. This method comprises combining a pharmaceutical agent with the biomaterial compound to a site of skeletal surgery, wherein such application results in controlled local release of the pharmaceutical agent.

The invention also provides a composition or a compound according to the invention for use as a medicament.

In another aspect of the invention, there is provided the use of a composition or compound according to the present invention, in the manufacture of a medicament for the treatment of bone related clinical conditions.

In another aspect of the invention, there is provided the use of a composition or compound according to the present invention in the manufacture of a medicament for substituting natural bone at sites of skeletal surgery in human and animal hosts; or for repairing large segmental skeletal gaps and non-union fractures arising from trauma or surgery in human and animal hosts; or for aiding the attachment of implantable prostheses to skeletal sites and for maintaining the long term stability of said prostheses in human and animal hosts; or for providing tissue-engineering scaffolds for bone replacement in human or animal hosts.

The biomaterial compound may be combined with additives such as those which increase the mechanical strength and toughness of the compound in order to provide additional functions for specific applications. The biomaterial compound may also be combined with various calcium phosphate materials such as calcium hydroxyapatite, α-TCP, β-TCP, octocalcium phosphate, tetracalcium phosphate, dicalcium phosphate and calcium oxide either as a physical mixture or as a solid solution.

The biomaterial compound has a distinguishable microporous and nanoporous structure along with a crystallography that is similar yet different from that of α-TCP. The new compound exhibits monoclinic pseudo-rhombic symmetry and is in the monoclinic space group P2₁/a. Furthermore, the new compound has a portion of the phosphorous substituted by an element having a suitable ionic radius.

The knowledge of the chemical formula of the biomaterial compound and the mechanism behind its bioactivity and stability in biological environments allows for the use of this compound *in vivo* for the treatment of various bone related clinical conditions. In particular, the compound may be used to help repair and restore natural bone that has been compromised by disease, trauma, or genetic influences.

### Brief Description of the Drawings

The present invention will be further understood from the following description with reference to the Figures, in which:
Figure 1 shows a X-Ray Diffraction Spectrum (θ-2θ) of powder prepared from the Ca-P colloid with no introduced additives and sintered at 1000 °C.
Figure 2 shows glancing angle XRD spectra of a thin film of the Ca-P colloid sintered on quartz at 1000 °C.
Figure 3 shows GA-XRD spectra illustrating the effect of sintering temperature on thin film phase composition;
Figure 4 shows GA-XRD spectra illustrating the effect of sintering time on thin film phase composition;
Figure 5 shows a SEM micrograph illustrating the characteristic surface morphology of a thin film of the Ca-P colloid sintered on quartz at 1000° C;
Figure 6 is a cross-sectional TEM of a Ca-P thin film on quartz, (a) film sintered at 1000°C (b) unsintered film;
Figure 7 shows the average agglomerate size in the Ca-P colloid as a function of colloid aging period, as determined using light scattering particle analysis;
Figure 8 shows a calculated predominance area diagram illustrating the effect of CaO activity on the relative stabilities of HA and TCP;
Figure 9 shows a θ2θ XRD spectrum of powder prepared from the Ca-P colloid with silicon as the introduced additive. Approximate phase ratio: 33±5% HA and 67 ±5%Si-TCP;
Figure 10 shows the effect of silicon content on phase composition of Si- mHA powders, as determined by x-ray diffraction (θ-2θ);
Figure 11 shows SEM micrographs illustrating the characteristic surface morphology of Si-mHA ceramic pellets. Si-mHA pellets can be resorbed by the specific cellular activity of osteoclasts in a manner similar to that which occurs on natural bone. (a) Surface morphology Si-mHA ceramic pellet; (b) Osteoclast lacunae on surface of Si-mHA ceramic pellet; and 11(c) Osteoclast lacunae on surface of natural bone;
Figure 12 shows θ-2θ XRD spectra of powder prepared from the Ca-P colloid with titanium as the introduced additive.
Figure 13 shows the effect of Ti addition on mHA phase composition; (a) no carrier (powder), (b) no carrier (ceramic pellet), (c) 2Me (powder), (d) 2Me (ceramic pellet), (e) ACAC (powder) and (f) ACAC (ceramic pellet);
Figure 14 shows SEM micrographs comparing the microstructure of Si-mHA pellets formed from the Ca-P colloid versus materials prepared from commercial sources. (a) Si-mHA prepared using TPOS as the introduced additive; and (b) cHA as a physical mixture with TPOS.
Figure 15 shows the XRD spectra for the physical mixture of 25% CaSiO₃ and 75% β-TCP sintered at 1250°C for 8 hours;
Figure 16 shows a high resolution XRD spectrum of Si-mHA powder;
Figure 17 shows the NMR spectra comparing Si-mHA with commercially available reference materials; (a) mixture of commercial CaSiO₃ and SiO₂ powders, (b) Si-mHA powder;
Figure 18 shows IR spectra for powders sintered at 1000°C: (a) cHA, (b) mHA and (c) Si-mHA; and
Figure 19 shows a summary of the IR spectra illustrating the effect of silicon content on the P-O stretch.

In the drawings, preferred embodiments of the invention are illustrated by way of example. It is to be expressly understood that the description and drawings are only for the purpose of illustration and as an aid to understanding, and are not intended as a definition of the limits of the invention.

### Detailed Description of the Preferred Embodiments

The Applicants have developed a process to provide a stabilized calcium phosphate synthetic biomaterial compound which is fully biocompatible and has a surface morphology capable of consistently supporting bone cell activity thereon. This is provided in accordance with that method described in the Applicant's co-pending published PCT application WO 97/09286, the subject matter of which is incorporated herein by reference. The preferred embodiment for making the compound of the present invention is described herein in the accompanying examples.

The compound of the present invention is herein referred to as a biomaterial compound due to its bioactive nature in both *in vitro* and *in vivo* systems. Bioactivity refers to the ability of the biomaterial compound to support osteoclast and osteoblast activity and the ability to be assimilated with natural bone by the activity of these cells. Although the compound was characterized with respect to the process by which it is made as well by its surface morphology, the molecular structure was unknown and could not be determined. It was however essential to characterize the compound further with respect to its chemical structure so as to better understand the properties of the compound as well as to understand why the compound was so well adapted for osteoclast and osteoblast activity. The knowledge of the chemical structure of the compound also allows for the modification of the compound for therapeutic use in the treatment of certain clinical conditions.

It was initially thought as described in WO 97/09286 that the compound was a silicon stabilized α-TCP. However, with further difficult and tedious analysis it was surprisingly found that the compound was in fact a completely new compound never before characterized and herein referred to as Skelit^{™}. Where silicon is used as the introduced additive to form Skelite^{™} the compound is referred to as Si-TCP. One reason for the great difficulty in establishing the chemical formula for the new compound was due to the complex and large structure of Ca-P compounds such as HA as well as the changing phase transitions that occurred during the sintering process. The chemical identification of this compound was only realized and developed after lengthy analysis of various Ca-P powders, thin films and pellets prepared with introduced additives in accordance with that described in WO 94/26872 and WO 97/09286. Consistent with that disclosed in applicant's international application WO 97/09286, standard XRD analysis was performed on samples prepared by a variety of compositional and thermal processing routes. Results were initially considered to be consistent with the conclusion that the materials were a mixture of α-TCP and HA, and that the calcium silicates predicted by the FACT database [23] existed as a glassy phase at the grain boundaries. Since no JCPDS file was available for Skelite^{™} and the peak positions were indicative of α-TCP using standard XRD techniques, the identification of Skelite™ was unforeseen. Furthermore, one would not expect to find that substitution is taking place at such low temperatures. A complex and unobvious combination of analysis techniques had to be performed to successfully identify and characterize the new compound. These studies, described as follows led to the characterization of the new compound, an additive stabilized calcium phosphate compound, Skelite^{™}.

For clarity, several materials referred to herein are defined as follows. For the commercially available materials cHA refers to commercial calcium hydroxyapatite (HA), calcium silicate refers to CaSiO₃ and silica refers to SiO₂. For the internally prepared materials mHA refers to microporous calcium hydroxyapatite (HA), Si-mHA refers to Si-TCP plus mHA. These materials are further defined in Table 1.

### Analysis of Pure (No Introduced Additives) mHA Powders

Using reaction (1) and analogous reactions, a fine colloidal precipitate of HA in ammoniated water can be achieved under conditions where the pH is greater than 10.

5Ca(NO₃)₂ + 3NH₄H₂PO₄ + 7NH₄OH → Ca₅(OH)(PO₄)₃ + 10NH₄NO₃ + 6H₂O (1)

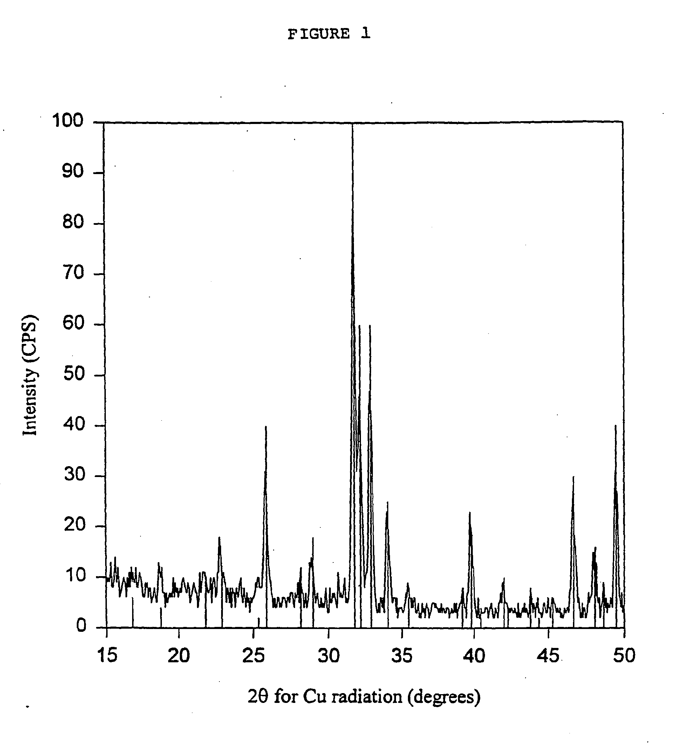

Figure 1 shows that powders prepared from the colloidal suspension of equation (1) with no introduced additives and sintered at 1000°C are HA (JCPDS File #9-432). The particle size of sintered powders, after mild grinding following sintering, is about 1 µm as determined by SEM.

### Analysis of Thin Films on Quartz Substrates

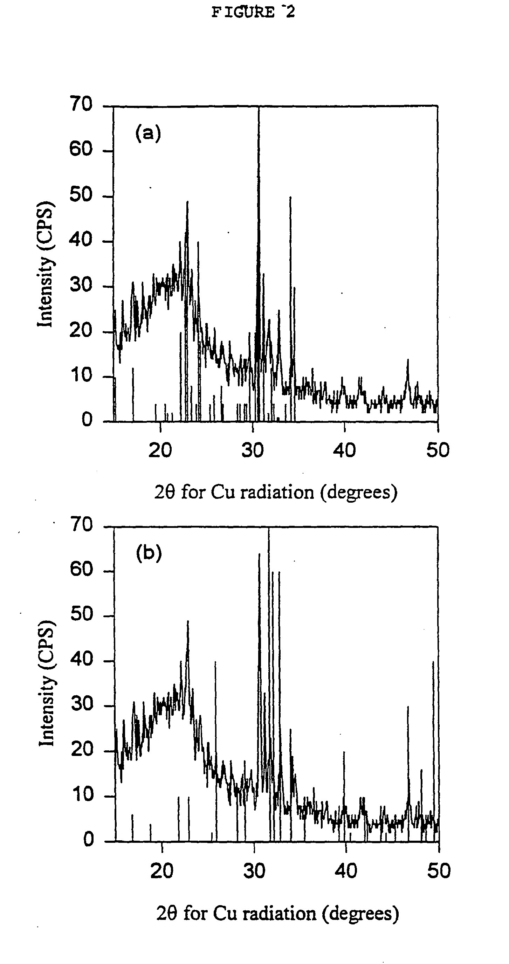

Figure 2 shows that the film on quartz has a crystallographic structure which was more complex than that for a powder sintered under the same conditions. The structure consists of two major phases, HA and Si-TCP, where the Si-TCP resembles, but is different from the crystallography of α-TCP (JCPDS file #9-348). All peaks within the XRD spectra could be attributed to either HA or Si-TCP and no distributions of peaks characteristic of other phases (such as β-TCP or octacalcium phosphate) were distinguishable from background.

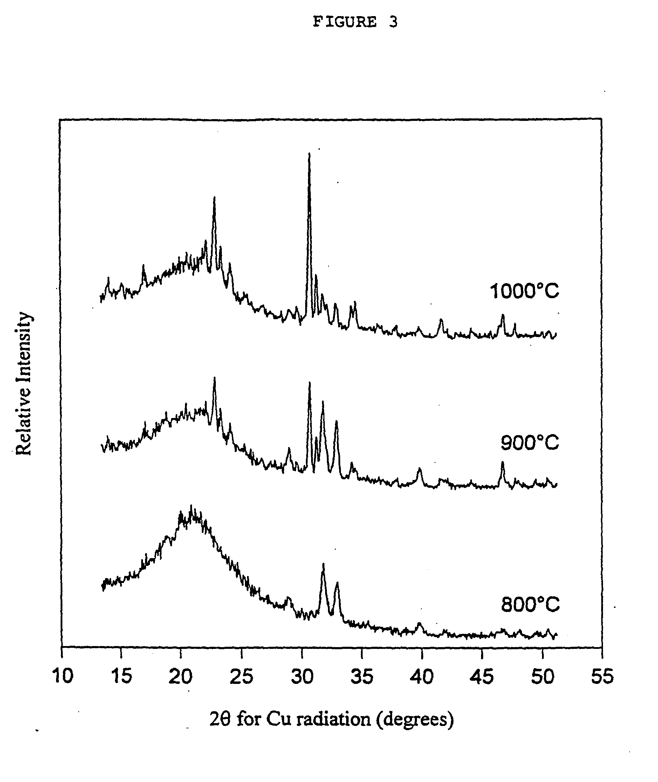

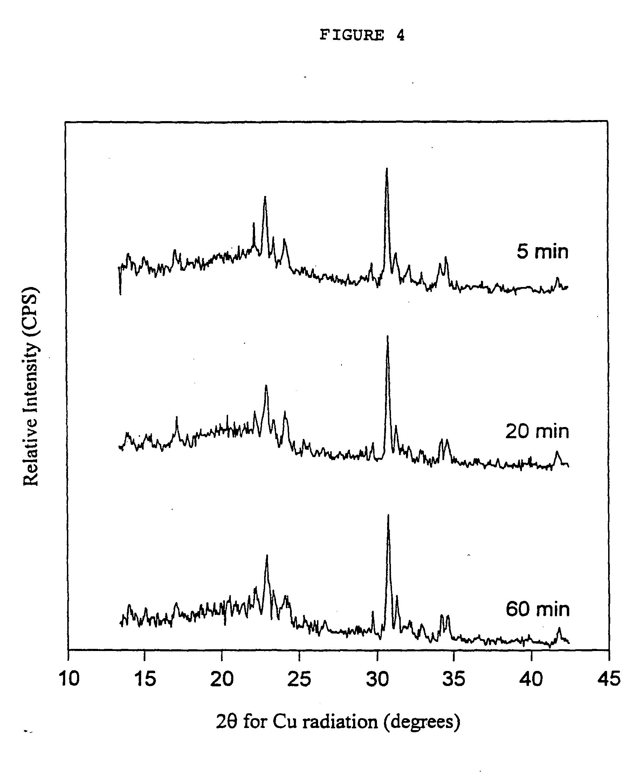

Figure 3 shows that as the sintering temperature was increased, the film composition changed. When the film was fired for one hour at 800°C the composition of the film was 94% HA and 6% Si-TCP; at 900°C there was a mixture of 62% HA and 38% Si-TCP; at 1000°C the composition was 33% HA and 67% Si -TCP. Changes in composition and film morphology as a function of sintering duration were assessed by changing the time the thin film on quartz remained in a furnace maintained at a set temperature. A computer controlled system allowed the ramp rate and hold temperature to be defined. Figure 4 shows that a dwell time of five minutes yielded the same equilibrium phase composition as observed after a one hour dwell time. Increased dwell time resulted in grain growth, as shown by SEM studies.

The phase composition could be modified by changing the humidity of the sintering environment while maintaining the firing conditions at 1000°C for one hour. The reaction was suppressed by the presence of increased water vapor. Other external factors or the addition of additives to the colloid suspension did not significantly modify the results achieved for thin films on quartz.

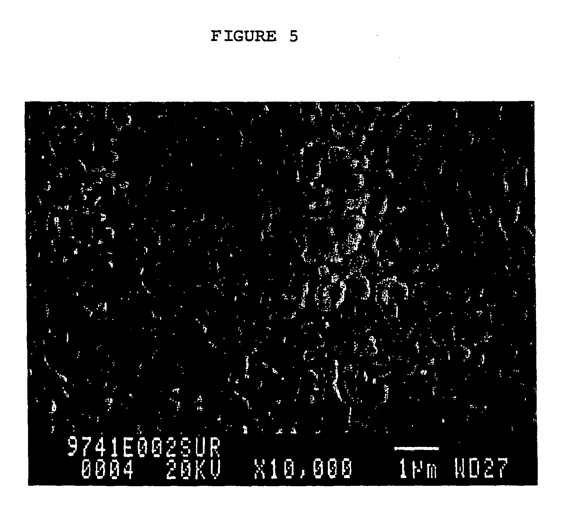

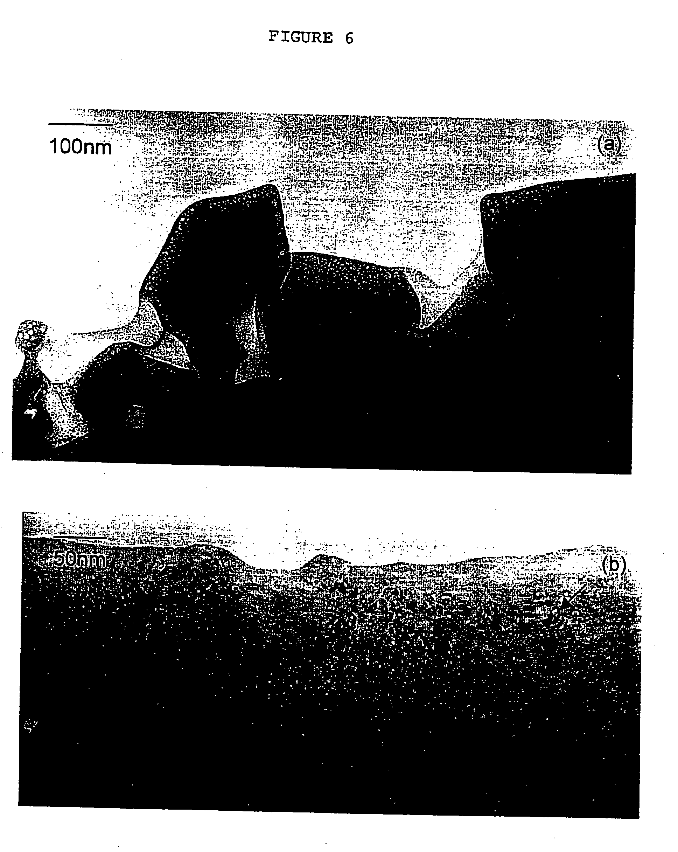

Optical microscopy, SEM and TEM show that the sintered films on quartz have a consistent morphology which is illustrated in Figures 5 and 6(a). While the films appear to be composed of translucent polycrystals under an optical microscope with phase contrast (x20), at the higher magnification achieved using an SEM (x10K) the surface morphology is that of an interconnected set of rounded particles with a high degree of porosity as seen in Figure 5. The average dimension of these particles depends on the time and temperature of sintering. Under most conditions the mean size lies between 0.2 and 1 µm with the size increasing with the time and temperature of sintering. Cross-sectional TEM (Figure 6(a)) of an individual particle indicates the presence of nanoporosity within the body of the particle. It is important to note that these pores were not altered with extended exposure to the electron beam, and were therefore inherent to the sample and not a sample preparation artifact. The underlying granular structure was about 5-10 nm in size. This appeared to reflect the individual granule size observed in the cross-sectional TEM micrographs of a dried but unsintered thin film on quartz as seen in Figure 6(b).

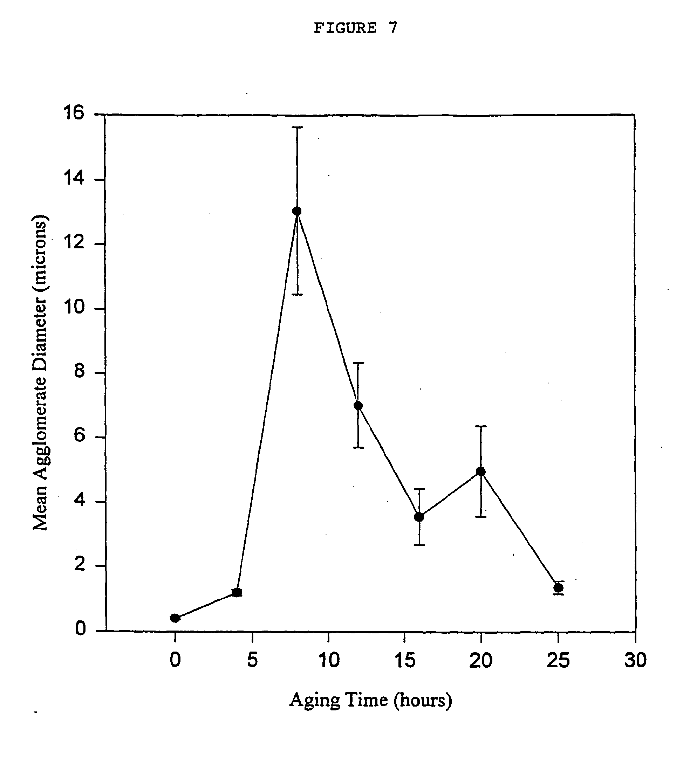

To examine the evolution of particle agglomeration, aliquots of the colloidal suspension were analyzed for particle size after various aging times. Figure 7 shows that a marked variation in measured particle size occurs during the 24 hour period of aging. The initial measurement gives a particle size less than 1 µm, increasing to greater than 10 µm after 8 hours, but subsequently decreasing again to approximately 1 µm after 24 hours. This is indicative of agglomeration of the fine precipitate with the most stable structure having dimensions in the range of 0.2-1.0 µm. Subsequent sintering of such agglomerates accounts both for the basic morphology of the thin films on quartz and the microporosity of bulk ceramics

In order to understand the origin of the difference between a precipitate fired as a powder or as a thin film prepared on a quartz substrate, films on quartz were fired for 1 hour and were subsequently analyzed for elemental composition as a function of distance from the quartz interface using electron induced energy dispersive X-ray spectroscopy (EDX). Silicon was detected at concentrations which decreased with distance from the interface; however, no XRD peaks for compounds such as calcium silicate could be identified. These results suggested that Si diffusing from the quartz substrate played a role in modifying the morphology and crystallography of the thin films.

### Analysis of mHA Powders with Introduced Additives

Powders prepared from the colloid of equation (1) combined with selective additives, demonstrate a unique calcium phosphate composition after sintering at 1000°C. Several possible actions of silicon as an additive in this temperature range were initially postulated such as the modification of the conversion reactions of HA into its successor compounds; the modification of the crystallographic structure of HA and its successor products by silicon substitution; and morphological changes associated with surface diffusion of the additive or by additive induced changes in surface properties.

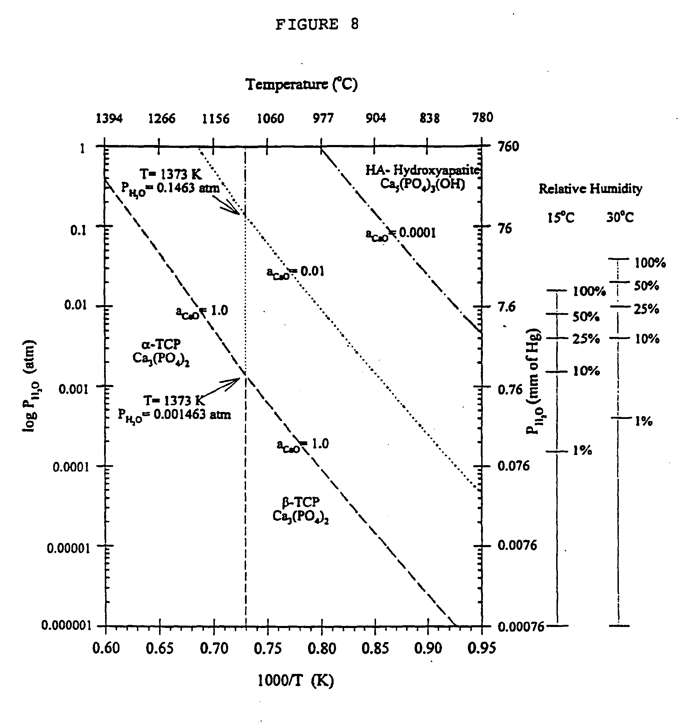

These possibilities were evaluated by the creation of ceramic thin films, powders and bulk materials in which processing conditions or the presence of additives changed the final products. The initial basis for defining process changes and additive selection was determined according to equilibrium thermodynamic computations using the database and programming in the Facility for the Analysis of Chemical Thermodynamics (FACT)[23]. Figure 8 shows the calculated phase diagram expected for the Ca-P system as a function of inverse temperature (K⁻¹) and partial pressure of H₂O in the thermal processing atmosphere. The diagram applies to a closed chemical system and utilizes a large database of literature values for the Gibbs free energies of formation. The most stable phase(s) are computed for a large matrix of coordinates which lead to the placement of the phase boundaries. HA decomposes into β-TCP at temperatures below 1100°C under low partial pressure of H₂O. α-TCP is formed at temperatures above about 1100°C. The predictions are consistent with high temperature crystallographic data for HA ceramics [24, 25]. The decomposition reaction, corresponding to the lowest diagonal line on the diagram, may be written as equation (2):

2Ca₅(OH)(PO₄)₃ ^{←}_{→} 3Ca₃(PO₄)₂ + CaO + H₂O (2)

Since the conversion of HA into TCP results in the simultaneous formation of CaO and release of H₂O, changes in the activity of both CaO and H₂O should modify the location of the phase boundaries. The upper diagonal lines show the phase boundary when the activity of CaO is made progressively smaller. This effect can be practically accomplished by chemical combination of CaO with other compounds such as SiO₂. In the presence of silica (SiO₂), the resultant compound could be one or more of several calcium silicates. The calculations show that the decomposition boundaries in the temperature range of 800-1100°C are in approximate agreement if CaO has the activity expected when the CaO is combined with SiO₂ as follows in equation (3):

CaO + SiO₂ ^{←}_{→} CaSiO₃ (3)

The most stable phosphorous-containing conversion product is, however, β-TCP. This is consistent with the widespread observation of the magnesium doped HA-based mineral whitlockite as the natural form of β-Ca₃(PO₄)₂ [25]. On the basis of the information available within the FACT database it is not possible to explain the observation of a phase similar to α-TCP as a conversion product below 1000°C other than to assume that β-TCP is not nucleated when CaSiO₃ forms and that Si-TCP develops as a metastable allotropic form.

It may be noted that on the basis of chemical thermodynamics, any reaction which changes the activity of CaO should modify the phase diagram. Oxides such as TiO₂ have only one product with CaO as in equation (4):

CaO + TiO₂ ^{←}_{→} CaTiO₃ (4)

and may therefore be more predictable in their action. Similar calculations to those for Si showed that for a similar partial pressure of water, the phase boundary for Ti was located at a slightly lower temperature.

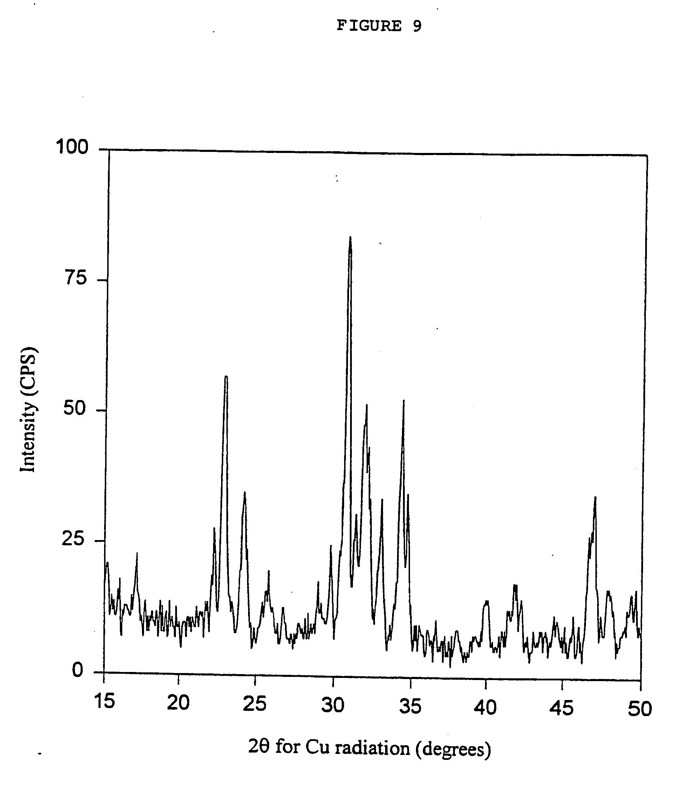

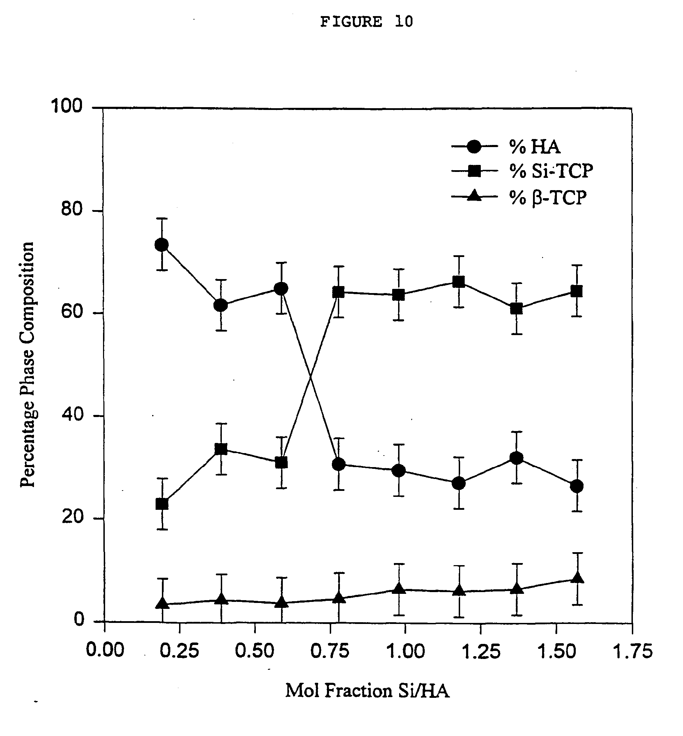

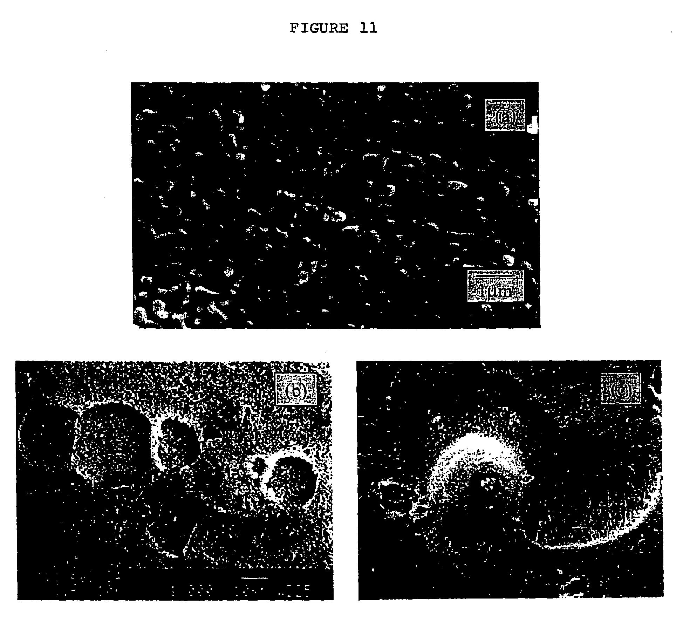

Figure 9 shows that the XRD pattern for a powder prepared using an additive concentration of 1 mol SiO₂ to 1 mol mHA is similar to that obtained for thin films on quartz. For this sample, the silicon was added as tetrapropyl orthosilicate in 2-methoxyethanol. The spectrum was compared to JCPDS files and concluded to be a mixture of HA and Si-TCP. Subsequent experiments demonstrated that the phase composition was independent of whether the additive was introduced with 2-methoxymethanol, 2-4 pentanedione or no carrier. Figure 10 shows the phase composition of powders sintered at 1000°C for one hour as a function of silicon content, as determined by XRD. The phases present switch from predominantly HA to predominantly a new compound (Si-TCP) at a relative molar Si/mHA ratio of approximately 0.6. The conversion is slightly greater when powders are formed into ceramic pellets. While the specific level of conversion is dependent on processing conditions, the typical Si-TCP:HA range is 20:80 to 80:20. Due to the increased signal to noise ratio and a more linear change of the background as a function of 2θ evident in the θ-2θ XRD spectra of the powders, the accuracy of determination of the phase composition in powders is increased. Additive saturation is evident at molar ratios exceeding 1:1 indicating process constraints in the integration of further silicon. Figure 11(a) shows that the crystalline morphology of a pellet formed from Si-mHA was similar to that observed in the thin films on quartz. The ceramic comprises rounded, inter-connected particles of average size 0.2 - 1.0 µm with a large degree of localized porosity. Varying the compound preparation condition permits the formation of a range of microporous structures comprised of particles of size range 0.1 to 2.0µm. Figure 11(b) indicates that Si-mHA materials show strong evidence of osteoclastic resorption similar to that which occurs on natural bone as shown in Figure 11(c).

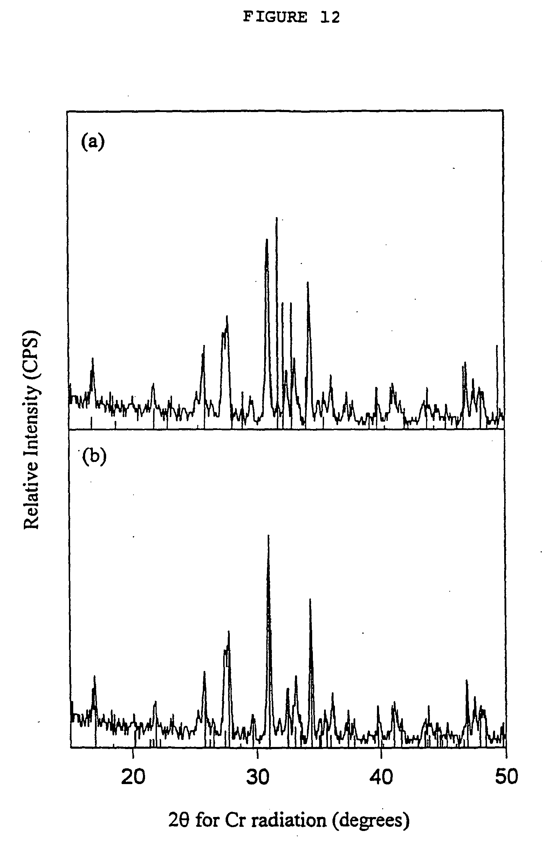

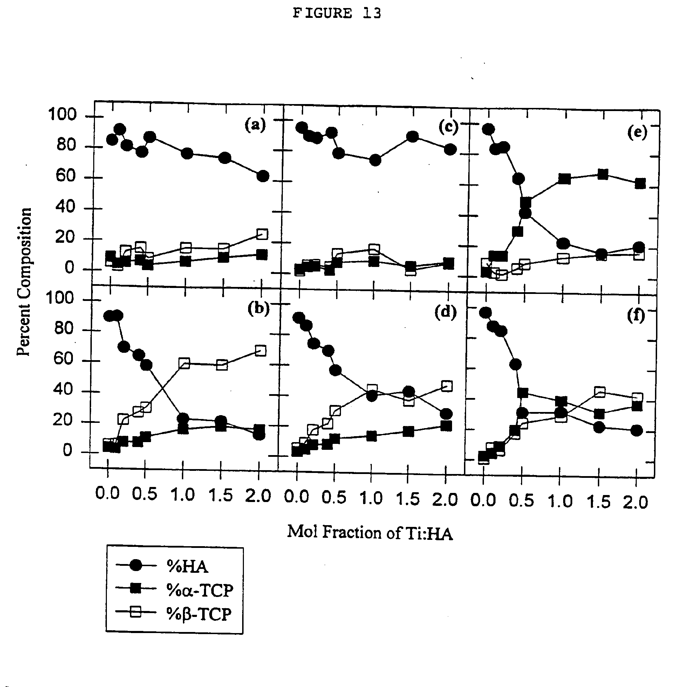

The XRD pattern for powders prepared using Ti as the additive also showed that conversion occurs upon addition of the Ti. However, the results were more complex as the predominant phase of TCP formed was β-TCP (Figure 12) with the degree of conversion dependent on the carrier used with the additive. Furthermore, there was enhancement of the degree of conversion on powder grinding and processing to form ceramic pellets. The results are summarized in Figure 13. Figures 13(a) and 13(b) respectively show the effects of titanium addition with no carrier for powders and ceramic pellets. Substantial conversion only occurs for pellets which were formed by grinding, pressing and resintering the original powder. The addition of titanium is similar or even less effective when 2Me is used as the carrier (Figures 13(c) and 13(d)). Substantial conversion at approximately 0.5 mol TiO₂ per mol mHA occurs in powders only when ACAC is used as a carrier and again this conversion occurs more effectively in the reground pellets, Figures 13(e) and 13(f). Particularly in the ceramic pellets, the phase composition shows a substantial fraction of β-TCP. The microstructure of pellets created from powders where Ti was the additive showed a particle size of approximately 0.3 µm.

The simplest interpretation of the differences between the effects of Si and Ti additives is based on the observation of the effects of additive precipitation and the changes observed in degree of conversion following powder grinding and pellet formation. In the case of Si-based additions, the degree of precipitation was essentially independent of the carrier and relatively minor changes in the degree of conversion occurred on formation into ceramic pellets. In contrast, Ti additions were ineffective when precipitation occurred when the additive was introduced into the Ca-P colloidal suspension (for no carrier and 2Me). Ti additions were effective when precipitation did not occur (for ACAC) and conversion became stronger upon grinding of the powder to form pellets and subsequent resintering. This suggests that the conversion from HA to TCP requires intimate contact between the additive and HA, possibly through surface functionalization of the precipitated mHA particles within the colloid suspension by the additive species or adsorption of the additive species on the surface of the mHA particle. When the additive and the mHA precipitate as separate species, the conversion occurs only upon strong physical inter-mixing and thermal treatment.

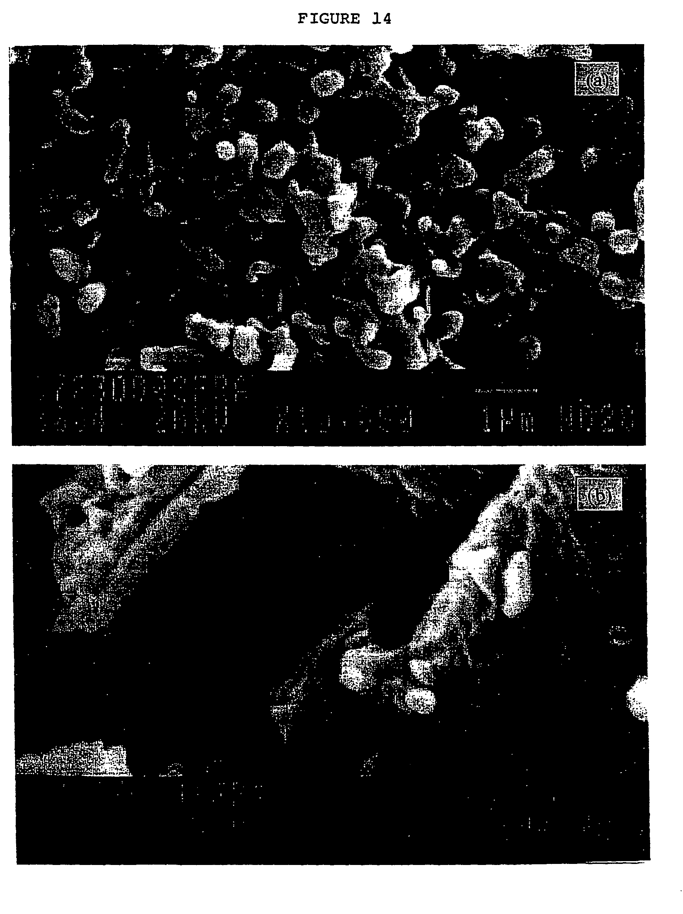

For comparative purposes, reference materials were prepared by equivalent thermal processing of commercially available powders (see Table 1) in an attempt to produce ceramics with a similar phase composition and surface morphology. Commercial powders were processed as pure compounds and in combination with selective additives introduced either as inorganic powders or as metallorganic species in a carrier. XRD results indicate that conversion of commercial HA (cHA) does take place, but that the primary resultant phase is β-TCP. The typical phase distribution is 73% β-TCP, 20% α-TCP and 7% HA. These results are consistent with the phase composition predicted by thermodynamics as noted in equations (2) and (3) and illustrated in Figure 8. Of equal significance is that the surface morphology of the ceramics prepared from these powders exhibits a jagged or fractured morphology (Figure 14b) with little inter-connectedness, and a particle size an order of magnitude greater than observed in colloid-based mHA pellets (Figure 14a). Evidence for a microporous morphology is restricted to the surface region of the particles. Pellets prepared in this fashion show no indication of resorption by osteoclasts.

The solid state chemistry of the cHA powders with introduced additives suggest that the conversion behaviour as a function of temperature, humidity and additive is consistent with equations (2)-(4). In particular, if physical mixing of the additive into the cHA powders takes place the β-TCP phase predicted by chemical thermodynamics is observed. In comparison, if intimate mixing of an unprecipitated silicon additive and a Ca-P colloid occurs the resultant phase is Si-TCP. This phase is not consistent with the predictions of equilibrium thermodynamics, but it is closely linked with the presence of Si in the Ca-P lattice. In order to use the FACT database to predict the phase boundaries for transitions to this Skelite^{™} compound, new values for the Gibbs free energy will be required.

The origin of the Skelite^{™} compound and confirmation of the mechanism of formation was investigated using techniques which assess the location of the additive within the HA or TCP structures, in an attempt to observe the presence of the reaction products predicted by equations (3) and (4).

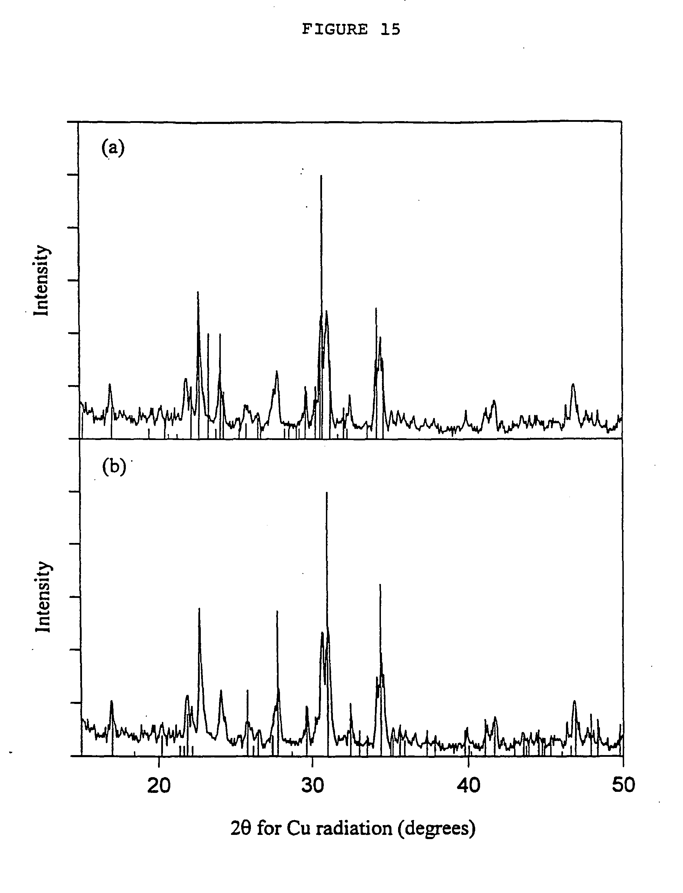

Significantly, no calcium silicate peaks were identifiable in XRD spectra taken on either colloidal-based or mixed powder compositions where Si was the selected additive. This suggests that Si forms a dispersed or substituted phase within the phosphate lattice. Previous workers [26, 27] have suggested that calcium silicate and β-TCP form a miscible solid solution at high temperatures (>1350°C) over the composition range of interest. The XRD spectra reported in these earlier experiments did not match that of α-TCP or the Skelite^{™} presently described, thus demonstrating the uniqueness of this compound. In this work, when commercially available CaSiO₃ was physically mixed with cHA or β-TCP powders (Table 1) and then sintered for 8 hr in alumina crucibles in air at 1250°C, the results showed that CaSiO₃ nucleates a crystallographic phase consistent with the Skelite^{™} compound (Si-TCP) (Figure 15). The degree of conversion to Skelite^{™} increases as the temperature of the reaction is increased. At 1250°C and above, depending on the amount of Si present, the powder mixtures show an increasing tendency to form a melt thus eliminating the microporous structure.

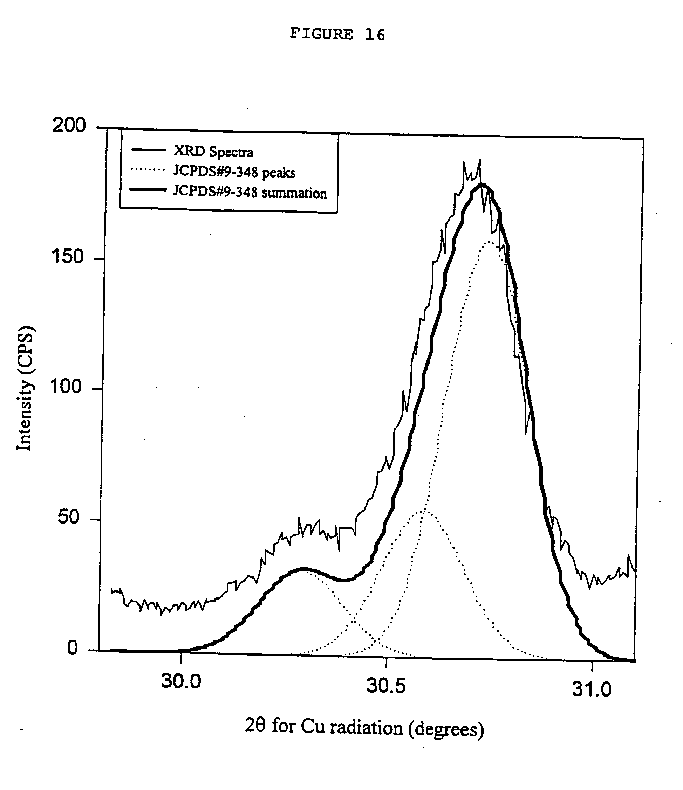

Comparison of three major peaks in the XRD spectrum of Skelite^{™} and α-TCP between 2θ_{Cu}=30 and 2θ_{Cu}=31°, assuming a Gaussian theoretical peak shape with a width of 0.225°, shows that there is a shift of approximately 0.1° to lower 2θ in Si-TCP (Figure 16) resulting from an increase in the lattice parameters. The presence of this significant shift was confirmed through the close examination of the position of the HA peak present in the XRD spectra. The HA peak, 2θ_{Cu}=31.8°, was within 0.01° of that predicted by the JCPDS file, and hence the accurate calibration of the instrument was assured. A peak shift in the α-TCP XRD spectra to lower 2θ would occur if Si⁴⁺ (IR=0.26 Å for CN=4) substitutes at P⁵⁺ (IR=0.17Å for CN=4) sites, although the effect would not be large since the lattice structure is dominated by the oxygen polyhedra of the TCP. The fact that the substitution reaction occurs at 1000°C only for colloidal particles in which the Si is chemically functionalized on the surface suggests that the substitution kinetics are very slow in the low temperature range.

### Nuclear Magnetic Resonance Studies

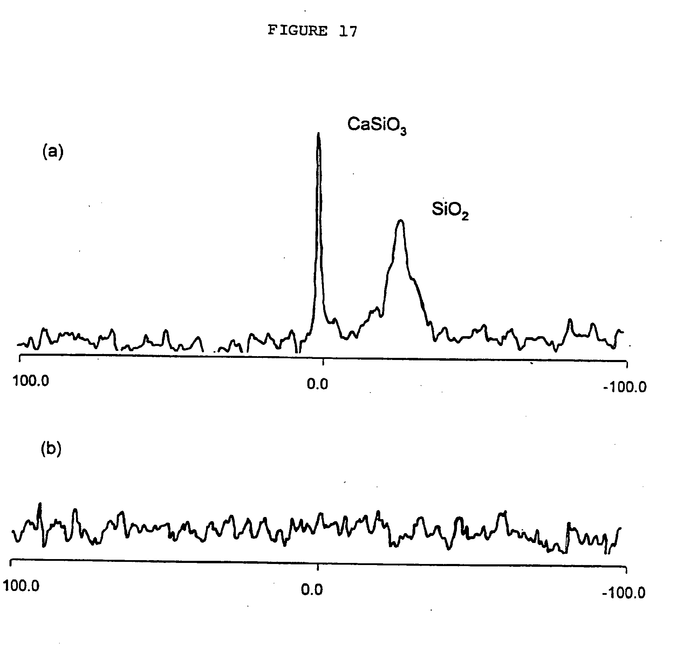

Magic-angle NMR studies were carried out on Si-mHA powders. Comparisons were made with simple physical mixtures of cHA, α- and β-TCP, CaSiO₃ and SiO₂ in proportions similar to the phases present in the Si-mHA powders. For Si-mHA, no Si signals could be observed under any conditions of measurement. Careful comparison with signals measured on CaSiO₃ and amorphous SiO₂ was used to set the lowest level of sensitivity at which the compounds or local structures could be measured. Figure 17 compares NMR spectra, signal averaged over 120,000 pulses, for Si-mHA with that obtained from a simple physical mixture of cHA and 10% of equal parts of CaSiO₃ and SiO₂. The absence of any NMR signal in the Si-mHA indicates that Si is highly dispersed throughout the crystallographic structure of mHA so that no clearly definable location or compound could be identified.

### Infrared Spectroscopy Studies

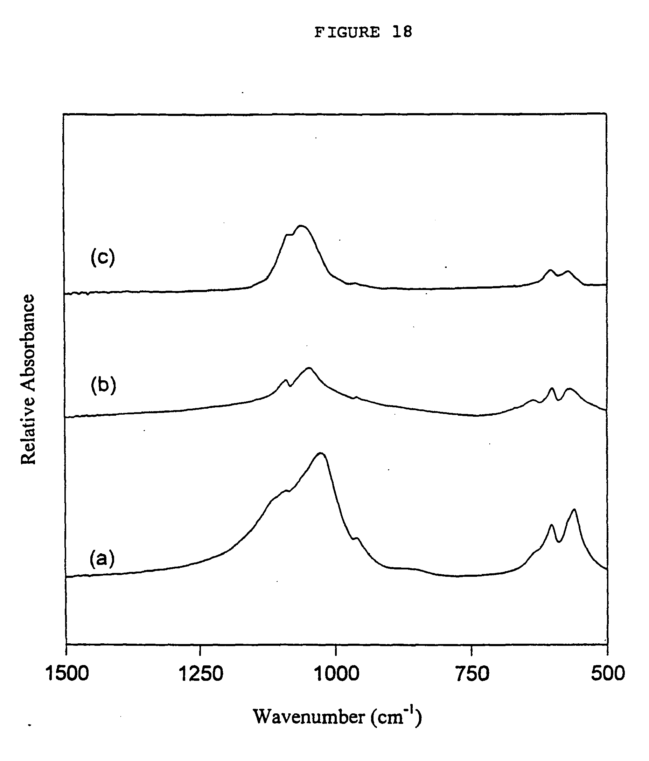

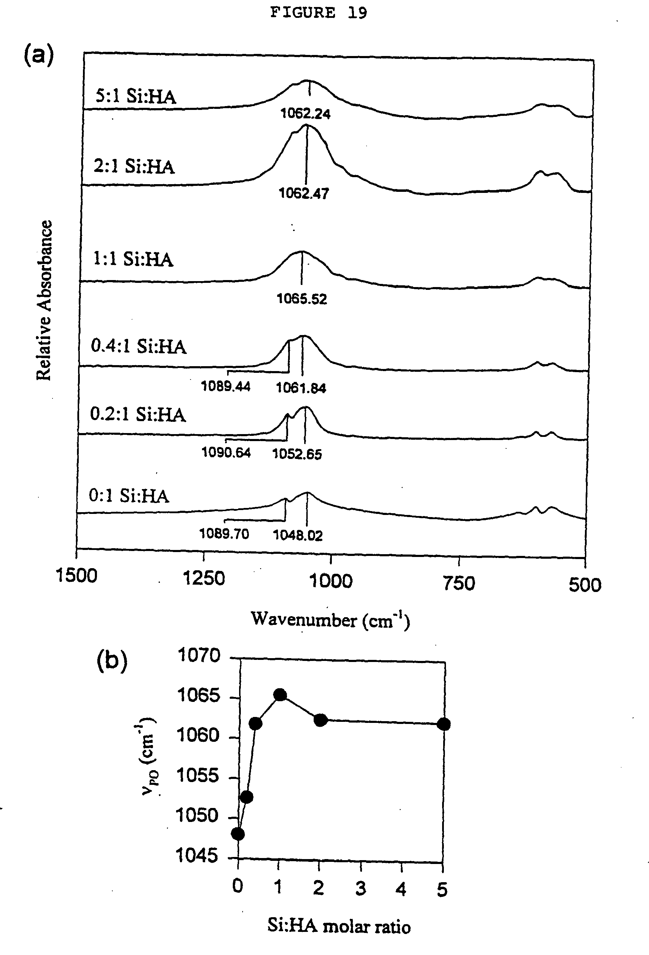

Figure 18 compares infrared spectra for sintered powders of (a) cHA, (b) mHA, and (c) Si-mHA. The peak pair found at the lowest wavenumbers near 600 cm⁻¹ indicate the presence of similar but not identical bonds. The spectra for cHA and mHA powders (no additives) were otherwise generally similar. Silicon addition causes a substantial narrowing of the P-O stretch peak and a shift in its position from 1048 to 1065 cm⁻¹ (Figure 19).

In order to assess these changes, IR spectra of CaSiO₃, CaO, SiO₂ and commercial β-TCP were examined. The CaSiO₃ spectrum shows a series of distinctive peaks at 717, 563 and 434 cm⁻¹ that are not apparent anywhere in the spectra for Si-mHA powders. The CaO spectrum has a strong sequence of bands below 463 cm⁻¹ which are also not observed in the Si-mHA spectrum. The SiO₂ spectrum shows a very strong, well-resolved peak at 1104 cm⁻¹ characteristic of the Si-O bond. An interpretation of the Si-mHA spectra is that the Si-O bond absorption occurs at lower wave numbers than in the pure SiO₂. The apparent shift in the P-O stretch can be explained by the growth of a Si-O peak. It is logical that the Si-O and P-O peaks would occur at similar positions since silicon and phosphorus are located beside each other in the periodic table and have similar ionic radii. The fact that the P-O peak appears to shift further indicates the formation of a new silicon compound, Skelite^{™}.

A structural model for silicon substitution based on the IR analysis is a crystal lattice of TCP-like and HA-like material with molecular dispersion of silicon throughout the lattice. This is consistent with the NMR and XRD results. The narrowing of the P-O peak suggests the existence of a less broad distribution of types of P-O bonds within the structure or an increase in crystallinity compared to the mHA with no introduced additives.

### The Skelite^{™} Compound

The significant correlations with cell-based bioactivity and resistance to dissolution at normal physiological pH 6.4 to 7.3 are the presence of the additive stabilized compound and the microporous morphology. The morphology is accounted for by the sintering of particles of average size 0.2 to 1.0µm. The presence of a Si-TCP phase that is essentially insoluble in biological media at low temperature using silicon as the introduced additive is unexpected and is induced by the distribution of Si substituted throughout the structure. Considering that the underlying structure of the particles is the agglomeration of granules of size range of approximately 1 to 20 nm, uniform dispersion of the silicon additive and functionalization of the surface of an individual granule is assured by permeation of the silicon sol throughout the agglomerate. The key aspect of this investigation was the determination that silicon does not induce an α-TCP phase resulting from the decomposition of HA, but rather it creates a Si-TCP phase, a new biomaterial compound, by substitution of silicon at phosphorus sites. The fact that silicon induces a Si-TCP compound can now be explained through the crystallography of the calcium-phosphate system and the defect chemistry associated with silicon substitution into the Ca-P lattice. One skilled in the art would understand that other additives having an ionic radius which is different to that of silicon as described herein, but may still substitute into the Ca-P lattice is also embodied for the compound of the present invention. Therefore the compound is not restricted only to silicon as the additive.

It is important to note that "effective ionic radius" has been selected as the term of reference in these studies [34]. The ionic radius specifications provided herein reflect the effective ionic radius for coordination numbers of 4, 6 or 8. It is apparent to those skilled in the art that "ionic crystal radius" may also be used in the practice of the present invention and thus may be used to define equivalent specifications for the compound and the formula of the compound as described herein. A summary of the effective ionic radius and the ionic crystal radius for various elements is provided in Table 2.

When substituting Si in the HA lattice, the ionic radius of Si⁴⁻ (IR=0.26Å for CN=4) suggests that Si⁴⁺ can enter at P⁵⁺ (IR=0.17Å for CN=4) sites within the PO₄³⁻ tetrahedra although it could also be included at Ca²⁺ (IR=1.0Å for CN=6) sites. The lattice strain and compensating defect will be significantly different in the two cases and the effects of covalency will substantially modify the result. A low temperature substitution of Si⁴⁺ into P⁵⁺ sites creates less strain and accommodates the covalency well. The radius ratio for silicon and oxygen is consistent with that required for the tetrahedral coordination of silicon in an oxygen lattice. Such a substitution requires the formation of a single positively charged defect for charge compensation. An obvious defect is one oxygen vacancy for every two silicon ions, although the energy required to displace oxygen-phosphorous bonds within an already formed PO₄³⁻tetrahedron may be substantial. Theoretically, substitution of an ion with an appropriate ionic radius and a valence of ≥3 at Ca²⁺ sites could also provide charge compensation. Such elements may include Ce, La, Sc, Y and Zr. Restrictions on the use of particular elements may be present due to the particular applications for use as a biomaterial.

In the formation of the Si-TCP compound, compositional analysis suggests that the Ca:P ratio decreases from approximately 1.67 (HA) to 1.5 (TCP). This could be induced by (1) the removal of calcium from the lattice, or (2) the introduction of additional phosphorous or an element that substitutes for phosphorous. A reduction in the calcium content of the lattice could theoretically occur by the formation of calcium silicate distributed within the structure. However, no evidence of calcium silicates as a well defined compound can be found in either the NMR or the IR results. Thus extensive silicon substitution must occur forming a multitude of Si-substituted P-O sites in the lattice.

In the case of Ti⁴⁺, the ionic radius of (IR=0.42Å for CN=4) likely precludes its substitution at P⁵⁺ sites and it must therefore enter the crystal at more general interstitial sites within the lattice. Since titanium has been demonstrated to be less effective in modifying the crystal structure to create a stabilized TCP, this suggests that the nucleation of the Si-TCP phase is intimately connected with the substitution of silicon at phosphorous sites. In particular, the observed phase being in fact a Ca-P-Si compound with a crystallographic structure similar but different from α-TCP rather than pure α-TCP, resolves conflicts with respect to the new compound's decreased solubility and the predicted decomposition phase diagram.

The crystallography of the Ca-P phase diagram has been extensively studied and compared [12] in apatites [28], β-TCP [29, 30] and α-TCP [31]. Significant differences have been noted between the structures of α and β-TCP [12, 31] and equally significant similarities have been seen between α-TCP, apatites and calcium silico-phosphate compounds via the glaserite structure [32]. A primary component of the phosphate lattice is the presence of PO₄³⁻ tetrahedra, although these structures can vary considerably throughout a complex lattice. For example, in α-TCP the P-O distances vary from 1.516 to 1.568 Å and the O-P-O angles vary from 104.1 to 115.2° [31]. Substitution of a Si at such sites implies a range of environments for such an additive.

Following Elliott [33] the space group of HA has three kinds of vertical or columnar symmetry. There are columns of Ca²⁺ ions spaced by one half of the c-axis parameter along three-fold axes which account for two-fifths of the Ca²⁺ ions in the structure. These ions are given the designation Ca(1). The Ca²⁺ ions are linked together by PO₄ tetrahedra in which three oxygen atoms come from one column and the fourth comes from an adjacent column. The result is a three-dimensional network of PO₄ tetrahedra with enmeshed Ca²⁺ ions, and channels that contain the residual calcium, Ca(2), and ions such as OH' which make up the HA structure.

The α-TCP structure also comprises columns of Ca²⁺ and PO₄³⁻ ions parallel to the c-axis [28]. The columns are actually anion-anion columns ...Ca Ca Ca Ca..... and cation-anion columns ... PO₄ Ca PO₄ □ PO₄ Ca PO₄ □ PO₄ Ca PO₄ .... where □ is a vacancy [12]. The presence of this vacancy may facilitate the creation of O²⁻ vacancies in neighboring PO₄³⁻ tetrahedra required to accommodate the substitution of Si⁴⁺ at P⁵⁺ sites. Analogous cation-anion columns occur in glaserite, K₃Na(SO₄)₂, except that the vacancy is occupied by a K⁺ ion. Strong similarities exist between the glaserite and apatite structures [26]. The apatite structure can be derived from that of α-TCP by replacing cation-cation columns at the corner of the apatite unit cell by anion columns (OH⁻ or F⁻). The remaining cation columns in α-TCP become the columnar Ca(1) ions in apatite, whilst the PO₄³⁻ and Ca²⁺ ions that form the cation-anion columns in α-TCP have approximately the same positions as the PO₄³⁻ and Ca(2) ions in apatite. Of significance to this analysis is that the glaserite structure is related to silico-carnotite Ca₅(PO₄)₂SiO₄ [30] and α-Ca₂SiO₄ [31]. This is consistent with the report that the system Ca₂SiO₄-Ca₃(PO₄)₂ forms a continuous series of solid solutions at higher temperatures based on the glaserite structure [27].

In contrast, there are no such similarities between the structure of HA and β-TCP. The β-TCP structure is a distortion of the parent lattice, Ba₃(VO₄)₂, with layers perpendicular to the c-axis. There is no columnar relationship between cations in the structure. Because of the size of the Ca²⁺ ion, there is a reduction in the number of PO₄ tetrahedra in the structure compared to that for the parent lattice and a reduction in the number of formula units within the hexagonal unit cell. Two types of Ca sites exist within the β-TCP unit cell: those known as Ca(5) are fully occupied, while a particular set of cation sites known as Ca(4) are only half occupied [12]. Upon doping TCP with Mg²⁺ (IR=0.72Å for CN=6) the Mg distributes itself first randomly on the Ca(4) and Ca(5) sites, but subsequently only substitutes at the Ca(5) sites. Because Mg²⁺ is smaller than Ca²⁺ (IR=1.0Å for CN=6) and the original distortion of the Ba₃(VO₄)₂ structure occurred because Ca²⁺ is smaller than Ba²⁺ (IR=1.35Å for CN=6), the β-TCP structure is stabilized with the addition of Mg²⁺ to form the naturally occurring mineral, whitlockite [31]. Indeed the addition of Mg to β-TCP at high temperatures tends to stabilize the structure well into the α-TCP range. In the case of the addition of an ion such as Ti⁴⁺, the slightly larger ionic radius (IR=0.61Å for CN=6) would suggest that it would also be accommodated by substitution at Ca(5) cation sites with results that are less defined than for Mg²⁺. Since charge compensating defects are necessary, the stabilization or creation of Ca²⁺ vacancies on Ca(4) sites would serve this purpose. Therefore substitutional Ti should stabilize the β-phase once TCP has been formed.

A feature of the presently characterized compound is that the Skelite™ structure is only achieved when intimate contact occurs between the precipitate and the additive. When silicon is introduced into already formed and fired powders at relatively low temperatures, the resulting post-sintered phase is predominantly β-TCP. In this case the silicon plays a role similar to that described for titanium above and simply acts to reduce the activity of CaO in the decomposition of HA under the terms of equation (3). In the case of colloidal powders precipitated in close association with an additive such as silicon, both the surface activity will be high and strongly functionalized complexes will be formed in the solution and at the interfaces of the precipitated granules. Through sintering, a range of PO₄³⁻ and SiO₄⁴⁻ tetrahedra will be established along with the necessary oxygen vacancies. In this case, nucleation of the glaserite-based Si/P phase will take place. While previously this was interpreted as a form of α-TCP it is, in fact, an entirely different compound with its own values for solubility and bioactivity (Si-TCP). Thus the crystal phase composition, surface morphology and bulk morphology originates from the chemically active and agglomerated state in which the starting material is precipitated, and the degree to which this state controls the location at which the Si⁴⁴ cation is substituted.

Again, although silicon has been the most extensively studied and appears to be the preferred substituted element of the invention, it is apparent to one skilled in the art, that any additive that can enter and distribute throughout the crystal structure of the calcium phosphate lattice and result in the compound of the present invention can be substituted for silicon. Therefore, the present compound is not restricted to only silicon as the substituted element but may also include other suitable elements having a suitable ionic radius of approximately 0.1-0.4Å such as for example boron. It is also understood that other additives in addition to the silicon or boron may also be present in the compound of the present invention. Such elements may also form part of the Ca-P lattice where such elements and/or the amount of oxygen may act to balance charge compensation for additives incorporated into the compound. Such additives may be selected from the group consisting of Ce, La, Sc, Y, and Zr.

It is also understood by those skilled in the art that the novel compound of the present invention can be combined with a calcium phosphate material such as calcium hydroxyapatite, α-TCP, β-TCP, octocalcium phosphate, tetracalcium phosphate, dicalcium phosphate, calcium oxide and other like materials. The resultant combination can be as a physical mixture or as a solid solution. In addition, other additives such as polymers or microfibers may additionally be added to the compound of the present invention to increase mechanical strength and toughness. The particle size of these additives may be selected such that the additive may be removed through phagocytosis by the action of macrophages. Metals may also be present in combination with the present compound to form composite structures. Such structures are also intended to be embodied in the present invention In summary, a new calcium phosphate-based biomaterial compound has been created and specifically characterized. This new biomaterial exhibits two prominent features:
(1) A unique composition created by the introduction of additives, such as silicon, into the colloidal precipitate to form upon sintering a stabilized calcium phosphate phase comprising the novel compound.
(2) A characteristic microporous morphology that arises from the agglomeration of particles within the colloid precipitate and the sintering of the material to produce a network of interconnected particles.

It is now revealed via numerous difficult analytical tests and complex data interpretation that this stabilized calcium phosphate compound is a novel additive stabilized structure referred to as Skelite^{™} that may exist in combination with HA, α-TCP, β-TCP or other suitable calcium phosphate phases. This new compound has been characterized to have the formula, (Ca_{1-w}A_{w})ᵢ[(P_{1-x-y-z}BₓC_{y}D_{z}Oⱼ)]₂, wherein A is selected from those elements having an ionic radius of approximately 0.4 to 1.1Å; B, C and D are selected from those elements having an ionic radius of approximately 0.1 to 0.4Å; w is greater than or equal to zero but less than 1; x is greater than or equal to zero but less than 1; y is greater than or equal to zero but less than 1; z is greater than or equal to zero but less than 1; x + y + z is greater than zero but less than 1; i is greater than or equal to 2 but less than or equal to 4; and j equals 4-δ, where δ is greater than or equal to zero but less than or equal to 1. The terms w and δ may be selected to provide charge compensation of the elements present in the compound.

An important processing step involves the intimate mixing of silicon as a candidate additive with the particles of the colloidal suspension to ensure the local availability of reactants. This in combination with the similarity of the silicon and phosphorous ionic radii, creates an environment favorable for silicon substitution at phosphorous sites within the Ca-P lattice and the development of the silicon-stabilized TCP structure.

The unique composition does not occur in the absence of intimate mixing as the effect of added silicon in these circumstances is only to influence the activity of CaO as an HA decomposition product. Similarly, the use of additives comprised of larger ions, such as titanium, cannot be accommodated in the lattice at phosphorous sites thereby precluding the important phosphate substitution phenomenon. In both of these cases, the resulting product is predictably β-TCP.

In view of the ability of Skelite^{™} to participate in the natural bone remodeling process, significant opportunities exist for the development of synthetic bone grafts and bone repair products that are indeed bioactive.

### Synthetic Bone Graft Applications

A synthetic bone graft that comprises in whole or in part the novel compound of the present invention has numerous applications in the orthopedic industry. In particular, there are applications in the fields of trauma repair, spinal fusion, reconstructive surgery, maxillo-facial surgery and dental surgery.

The gold standard in the industry for treating traumatized bone is an autologous bone graft, commonly referred to as an autograft. Autograft transplants involve a surgical procedure in which healthy bone is taken from an alternate part of the patient's skeleton to repair areas of skeletal trauma. Autografts however, require double surgical procedures; one for graft removal and a second for re-implantation at the damaged site. This makes the procedure very expensive and time consuming. Additionally, it is not uncommon for patients to subsequently suffer chronic pain at the autograft harvest site.

Another widely used bone graft technique is the use of allograft, a term referring to a tissue graft from another individual or animal. In this situation, bone is removed from the donor and implanted in the patient. Allografts are susceptible to various negative consequences. For example, the use of allograft from an animal other than a human carries the possibilities of cross species infection and immunological rejection. Even human sourced allograft, which is used more often than animal tissue, exposes the implant recipient to the possibilities of rejection and disease.

The use of Skelite^{™} eliminates the pain and costs associated with the bone harvest procedure required in autograft transplants. Furthermore, since Skelite™ is generated in a laboratory and is completely synthetic, it removes the possibility of transmission of infection and disease, as well as eliminates sources of immunological rejection by the patient.

Skelite^{™} fulfils the need for a versatile bone reconstruction material. Its ability to immediately stimulate local natural bone growth provides stability and rapid integration, while the body's normal cell-based bone remodeling process slowly resorbs and replaces the implant with natural bone. This removes the concerns of long term compatibility and durability associated with current artificial implant technologies.

Products formed from Skelite^{™} will involve different configurations in order to address the requirements of particular applications. For example, Skelite^{™}-based products can be manufactured as a fine or coarse powder, pellets, shaped three dimensional pieces, macroporous structures, thin films and coatings. In addition, these products could potentially carry an integrated bone growth factor to speed short term recovery.

The use of Skelite^{™} in a macroporous configuration allows the open porous structure to serve as a scaffold for the integration of new bone tissue. The macroporous structure is formed by the coating of the compound onto a reticulated polymer and subsequently removing the polymer through pyrolysis. The macroporous structure comprises an open cell construction with interconnected voids having a a pore size of approximately 50 to 1000 micron. Due to this design, Skelite^{™} is the ideal bone substitute for implantation at defect sites where special measures are required to encourage new bone growth to bridge areas of major tissue loss due to trauma or surgical intervention. The Applicant has identified two primary approaches for the clinical use of such a product: direct implantation and tissue engineering.

### Direct Implantation

The simplest approach is to directly implant the Skelite^{™} scaffold at the location of skeletal trauma where the bioactive properties of the biomaterial compound stimulate the body's natural bone repair mechanism. Once the initial healing process is complete, the Skelite^{™} scaffold is progressively replaced with natural bone as part of the body's orderly remodeling process.

Hybrid versions of Skelite^{™}-based products are possible where bone growth factors are incorporated into the scaffold as a post-manufacturing process or at the time of surgery. The availability of the growth factor at the repair site increases the rate of new bone formation thereby improving patient recovery time and lowering overall health care costs.

### Tissue Engineering

The concept that underlies the tissue engineering application is to remove bone cells from the patient's skeleton using an established bone marrow aspiration technique, and then carefully introduce the collected cells (cell seeding) into the open cell structure of the Skelite^{™} scaffold in a sterile biotechnology facility. The cells and scaffold are then incubated so that the cells have an opportunity to multiply and begin to fill the scaffold with new mineralized matrix. After several weeks, the biological implant is ready for implantation back into the patient. This biotechnology bone growth process is termed "tissue engineering", and the procedure serves to enhance the ability of surgeons to reconstruct severely compromised areas of the skeleton. Once successfully integrated at the repair site, the Skelite^{™} implant is subsequently remodeled into natural bone by the ongoing activity of bone cells.

A refinement of this approach is to selectively extract and grow in cell culture only special precursor cells termed Mesenchymal Stem Cells (MSCs). In order for these cells to remain healthy during biological processing, they need to be attached to a suitable physical carrier. In addition, the performance of the cells benefits from the addition of organic bone growth factors. Skelite^{™} is a suitable carrier since it allows for both the integration of bone growth factors and the attachment of specialized MSCs. In addition, following implantation and patient recovery, the Skelite^{™} scaffold is subsequently remodeled into natural bone.

The use of Skelite^{™} in direct implantation or tissue engineering applications has important advantages over the use of naturally sourced bone graft material, and consequently Skelite^{™} products have the potential to replace the autograft procedure as the orthopedic surgeon's preferred treatment strategy.

The key advantages of implantable products formed from the Skelite^{™} material are:
- Immediately stimulates local natural bone growth at the implanted site, thus providing early stability and full integration.
- Ensures long term biocompatibility and efficacy.
- Acts as a bioactive scaffold for use in advanced tissue engineering applications.
- Eliminates the cost and chronic pain associated with the double surgical procedures required in traditional autograft transplants.
- Eliminates the risks of immunological rejection and infection transmission.
- Meets the needs of various orthopedic applications, as the product is available in different configurations.
- Allows for the use of growth factors that can further increase the rate of natural bone healing and subsequent remodeling.
- Provides a means for timed-release drug delivery.
- Disappears naturally through the body's bone remodeling process once therapeutic function is complete.

### Drug Carrier Application

The Skelite^{™} biomaterial may also be used for the incorporation of selected pharmaceuticals into the compound for the further enhancement of the bone healing and remodeling processes. In this respect, pharmaceuticals that have been incorporated into the Skelite^{™}-based products can be predictably released at the site of implantation and hence become available to assist in the bone regeneration process. The Skelite^{™} biomaterial may also be designed as a slow release vehicle for appropriate pharmaceutical compounds.

Primary candidates for incorporation into Skelite^{™}-based products are selected bone growth factors. These proteins have been identified as being critically important in growing and maintaining healthy bone tissue. In particular, when applied at the site of traumatized bone, natural bone growth is enhanced with a corresponding improvement in overall therapeutic response. However, a compatible carrier system is required to deliver such therapeutic biologicals to the site and ensure local release of appropriate concentrations of the drug. Implant studies have shown that products formed from the Skelite^{™} biomaterial are suitable for use as drug carriers. One skilled in the art would understand that other pharmaceuticals such as antibiotics for example which may aid in the bone healing process may also be incorporated into the Skelite^{™} compound.

### Coating Applications

Through a liquid application process, the Skelite^{™} material can be coated on to orthopedic and dental implants to improve and promote natural bone fixation and to improve long term implant stability. Such a coating of approximately 0.1 to 10µm acts at the interface with the patient's own tissue to promote natural bone growth during the weeks immediately following surgery, and is then progressively replaced by the ongoing activity of bone cells once the initial healing process is complete. The result is a strong union between the implant and the host bone. This is not the case with conventional calcium phosphate implant coatings where the biologically inert coating is subject to mechanical detachment (delamination) from the metal substrate, causing potentially catastrophic implant failure.

The key advantages of an implant coating formed from the Skelite^{™} material are:
- Promotes rapid natural bone growth during the recovery period and is then progressively replaced through the body's orderly remodeling process.
- Eliminates the coating as a potential source of long-term failure and reduces the risk to the patient of incurring complicated and costly revision surgery.
- Reduces patient recovery time and associated health care costs.
- Permits a strong union directly between the implant and the patient's natural bone.
- Involves a manufacturing process based on a liquid application procedure which allows full coverage of the device, including complex surface geometries.

### Examples

The examples are described for the purposes of illustration and are not intended to limit the scope of the invention. The examples exemplify aspects of the invention for providing a Skelite^{™} compound which is an additive stabilized structure having unique physical characteristics and is fully biocompatible with natural bone tissue.

Methods of synthetic chemistry and organic chemistry referred to but not explicitly described in this disclosure and examples are reported in the scientific literature and are well known to those skilled in the art.

### Example 1 Preparation of Ca-P Colloidal Suspension (Sol-Gel)

A calcium nitrate solution was created by dissolving 4.72 g of Ca(NO₃)₂ in 80 mL of a solution of DDH₂O containing approximately 3 mL of 30% NH₄OH. Similarly, an ammonium phosphate solution was prepared by dissolving 1.38 g of NH₄H₂PO₄ in 192 mL of a solution of DDH₂O containing approximately 71 mL of 30% NH₄OH. The pH of the final solutions was approximately 11. The ammonium phosphate solution was added drop-wise into the calcium nitrate solution to form a calcium phosphate precipitate. On completion of the reaction, the solution and precipitate were aged for a period of 24 hours. Following aging, 240 mL of the solution containing the precipitate was centrifuged for 20 minutes at 500 rpm. Without disturbing the sediments, 180 mL of supernatant was discarded from the bottle. The sediments were then re-suspended by rotating the bottle on an orbital shaker for one hour.

The resulting Ca-P colloidal suspension may be used in a variety of further preparations.

### Example 3 Preparation of Ca-P Powder with No Introduced Additives

Following the procedures for the formation and aging of the colloidal suspension of Example 1, the colloid was processed to the stage of reducing the volume by centrifugation. The precipitate was dried for approximately 5 hours at 100°C and sintered for one hour in an open alumina crucible in air at a temperature of 1000°C. A fine powder was formed through mechanical grinding of the sintered material in a motorized mortar and pestle (Retsch Model RM 100 USA).

### Example 4 Preparation of Ca-P Powder with Silicon as the Introduced Additive

Following the procedures for the formation and aging of the colloidal suspension of Example 1, the colloid was processed to the stage of reducing the volume by centrifugation. In order to retain the colloidal sol characteristics, the silicon additive was introduced as a sol-gel metal-organic precursor in an organic carrier. The precursor was either tetrapropyl orthosilicate (Si(OC₃H₇)₄ or TPOS) or tetraethyl orthosilicate (Si(OC₂H₅)₄ or TEOS). Addition was accomplished by creating a sol using a precursor carrier such as 2-methoxyethanol (CH₃OCH₂CH₂OH or 2Me) or 2-4 pentanedione (CH₃COCH₂COCH₃ or ACAC). The action of the carrier was to ensure that the additive did not precipitate upon addition to an aqueous solution having a pH similar to that of the Ca-P colloidal suspension. This ensured that the additive was uniformly mixed within the colloid to create a single precipitate rather than two distinct precipitates. Precipitation of the additive was examined in a separate experiment with aqueous solutions. For the silicon compounds, precipitation was minimal for 2Me, ACAC and even if no carrier was employed. The precipitate with introduced silicon was dried for approximately 5 hours at 100°C and sintered for one hour in an open alumina crucible in air at a temperature of 1000°C. A fine powder was formed through mechanical grinding of the sintered material in a motorized mortar and pestle (Retsch Model RM 100 USA). The presence of the additive within the sintered ceramics was checked by wet chemical analysis.

### Example 5 Preparation of Ca-P Powder with Titanium as the Introduced Additive

Following the procedures for the formation and aging of the colloidal suspension of Example 1, the colloid was processed to the stage of reducing the volume by centrifugation. In order to retain the colloidal sol characteristics, the titanium additive was introduced as a sol-gel metal-organic precursor in an organic carrier. The precursor was titanium n-propoxide (Ti(OC₃H₇)₄). Addition was accomplished by creating a sol using a precursor carrier such as 2-methoxyethanol (CH₃OCH₂CH₂OH or 2Me) or 2-4 pentanedione (CH₃COCH₂COCH₃ or ACAC). ACAC was used in particular for its strong chelating action. Precipitation of the additive was examined in a separate experiment with aqueous solutions. For titanium n-propoxide, precipitation of the additive occurred for both no carrier and 2Me, but not for ACAC. The precipitate with introduced titanium was dried for approximately 5 hours at 100°C and sintered for one hour in an open alumina crucible in air at a temperature of 1000°C. A fine powder was formed through mechanical grinding of the sintered material in a motorized mortar and pestle (Retsch Model RM 100 USA). The presence of the additive within the sintered ceramics was checked by wet chemical analysis.

### Example 6 Preparation of Ceramic Pellets

Ceramic pellets were formed from previously sintered powder that had been prepared according to Examples 3, 4, or 5, using a small amount of the concentrated colloid suspension mixed into the sintered powder as a binding agent. The powders were uniaxially pressed into pellets with a pressure of 1x10⁸ N/m² [15,000 psi]. The final pellets were sintered for one hour in air at a temperature of 1000°C to create ceramic components with the desired characteristics. Following thermal processing, the pellet density was approximately 1.5 g/cm³, and the pellet exhibited a uniform microporosity throughout the structure.

### Example 7 Preparation of Macroporous Structures

Sintered powder that had been prepared according to Examples 3, 4, or 5, was sieved using a motorized sieve shaker (Retsch Model AS200 BASIC USA). Powder having a particle size of -325 Mesh was collected and subsequently suspended in water to form a slurry. The interior and exterior surfaces of a preformed piece of open cell (reticulated) polyurethane foam were completely coated by immersing the foam in the slurry. The slurry-coated component was then allowed to dry and was subsequently sintered at 1000°C for 1 hour. During thermal processing, the foam was removed from the structure through pyrolysis. Importantly, the shape of the final ceramic component replicates the original shape of the foam, including the open-cell structure.

In the preparation of these components, the pore density of the foam was selected to produce the required pore size in the ceramic. Typical pore sizes prepared were in the range of 45 to 80 pores per inch. The coating of the foam was managed to ensure complete coverage of the foam without clogging of the cells. The duration and temperature of the thermal processing were selected to ensure pyrolysis of the foam and to obtain the desired physical properties of the resulting macroporous structure.

### Example 8 Preparation of Drug Carrier with Associated Pharmaceutical Agent

Depending on application requirements, either the powder of Example 4 or the macroporous structure of Example 7 was sterilized using ethylene oxide or similar approved medical device sterilization technique. In a laminar flow hood, a liquid drug volume was made up according to dosing requirements. In the case of the agent BCSF^{™} (Bone Cell Stimulating Factor), this required addition of sterile normal saline (0.9 % NaCl) to previously lyophilized stored aliquots of the drug, at room temperature. Following reconstitution, the drug was either mixed by gentle agitation with the powder, or slowly dispensed over the surface of the macroporous structure.

Recognizing the natural protein avidity of the bioceramic material, a period of 5 minutes was allowed for the drug to percolate and bind to either the powder or the macroporous structure. Following this period, the preparation was ready for direct patient administration as a therapeutic device or for use as a tissue-engineering scaffold.

In the case of therapeutic administration of the powder-based preparation, a predetermined volume of the suspension (powder plus attached pharmaceutical agent) was injected percutaneously at the desired skeletal site.

In the case of therapeutic administration of macroporous structures, surgical intervention was required to implant the device at skeletal sites in order to effect subsequent bone repair.

### Example 9 Commercial Reference Materials

The commercially available HA (cHA), α-TCP, β-TCP, calcium silicate and silica materials listed in Table 1 (below) were used as reference standards for the analytical techniques performed in the evaluation of the internally prepared mHA and Si-mHA materials described in this study.

| Table 1: List of Materials Used for Experimental Samples and Reference Standards | | |
|---|---|---|
| ***Commercial Materials*** | | ***Source*** |
| Commercial HA | cHA | Aldrich #28,939-6 Lot#043021 Q |
| α-TCP | α-TCP | Supelco Inc #3-3910 Lot#200792 |
| β-TCP | β-TCP | Fluka #21218 Analysis#357352/1 14996 |
| Calcium silicate | CaSiO₃ | Aldrich #37,266-8 Lot#00714LN |
| Silica | SiO₂ | PPG Industries Inc. #63231674 Lot#9-134 |

| ***Internally Prepared Materials*** | | ***Preparation Technique*** |
|---|---|---|
| Microporous HA | mHA | Powder prepared from the thermal processing at the colloid in equation (1) |
| Si-TCP + mHA | Si-mHA | Powder prepared from the thermal processing of the colloid in equation (1) where Si is the introduced additive |

### Example 10 Analytical Techniques

X-ray diffraction (XRD) spectra of thin films were acquired using a glancing angle (GA-XRD) technique with an angle of incidence θ = 2°, whereas powders were examined using conventional θ-2θ geometry. The source was a 12 kW Rigaku rotating anode XRD generator fitted with a Cr target for improved peak resolution. The glancing angle geometry significantly reduced the contribution from the substrate. For convenience of comparison to other literature, all spectra were converted to that expected for a Cu anode using the following relationship: sin(θ_{Cu}) = (λ_{Cu}/λ_{Cr})sin(θ_{Cr}), where λ_{Cu} = 1.54056 Å and λ_{Cr} = 2.28970 Å. The phase composition was determined by comparing acquired spectra with peaks identified in the Joint Committee on Powder Diffraction Standards (JCPDS) database of standards [20]. Of particular relevance to this study are the XRD spectra of HA (JCPDS #9-432), α-TCP (JCPDS #9-348) and β-TCP (JCPDS #9-169). Following the collection of XRD data, the background noise was subtracted and the integrated intensities of peaks distinguishable as HA, α-TCP or β-TCP were calculated. These values were then used to determine the percentage phase composition (plus or minus 5%).

Optical microscopy, scanning electron microscopy (SEM, using a JEOL JSM 840) and transmission electron microscopy (TEM, using a Philips CM20) were performed to assess the surface and bulk morphology. Chemical analysis of the samples was carried out by wet chemical methods and neutron activation analysis. Wide-line nuclear magnetic resonance (NMR) experiments on ²⁹Si were accomplished using a Bruker NMR CXP 200 MHz spectrometer with magic angle spinning using a pulse width of 5 ms and a pulse delay of 20 s. Infrared spectroscopy (IR) of powders using a KBr pellet technique utilized a BOMEM MB-120 spectrometer. Approximately 2 mg of sample and approximately 200 mg of KBr were ground and pressed in a 6 mm diameter die at 10 tonnes for 1 minute to produce uniform discs for analysis.

A particle size analysis of the Ca-P colloid at various stages of processing was made by observation of 633 nm He-Ne laser light scattered at various angles. Samples were prepared by adding 10 drops of the precipitated solution to 4 mL ammoniated water (one part 30% NH₄OH mixed with five parts water) having a pH greater than 10. Results from these suspensions were reproducible for equivalent samples and stable over time. The power spectrum of the scattered light at a known angle was fitted to a Lorentzian distribution and analyzed by standard methods using a solution viscosity of 8.9 x 10⁴ kg m⁻¹s⁻¹ and refractive index of 1.3312 [21,22].

Although preferred embodiments have been described herein in detail, it is understood by those skilled in the art that variations may be made thereto without departing from the scope of the invention as defined by the appended claims.

### Table 2: Summary of Effective Ionic Radius and Ionic Crystal Radius for Various Elements

| Data from: Shannon, R.D., Acta Cryst. (1976) A32, 751 | | | |
|---|---|---|---|
| ***Ion*** | ***Coordination Number (CN)*** | ***Ionic Crystal Radius (CR)*** | ***Effective Ionic Radius (IR)*** |
| B³⁺ | 4 | 0.25 | 0.11 |
| | 6 | 0.41 | 0.27 |
| Ba²⁺ | 6 | 1.49 | 1.35 |
| | 8 | 1.56 | 1.42 |
| Ca²⁺ | 6 | 1.14 | 1.00 |
| | 8 | 1.26 | 1.12 |
| Ce³⁺ | 6 | 1.15 | 1.01 |
| | 8 | 1.28 | 1.14 |
| La³⁺ | 6 | 1.17 | 1.03 |
| | 8 | 1.30 | 1.16 |
| Mg²⁺ | 4 | 0.71 | 0.57 |
| | 6 | 0.86 | 0.72 |
| | 8 | 1.03 | 0.89 |
| P⁵⁺ | 4 | 0.31 | 0.17 |
| | 6 | 0.52 | 0.38 |
| Sc³⁺ | 6 | 0.89 | 0.75 |
| | 8 | 1.01 | 0.87 |
| Si⁴⁺ | 4 | 0.40 | 0.26 |
| | 6 | 0.54 | 0.40 |
| Ti⁴⁺ | 4 | 0.56 | 0.42 |
| | 6 | 0.75 | 0.61 |
| | 8 | 0.88 | 0.74 |
| Y³⁺ | 6 | 1.04 | 0.90 |
| | 8 | 1.16 | 1.02 |
| Zr⁴⁺ | 4 | 0.73 | 0.59 |
| | 6 | 0.86 | 0.72 |
| | 8 | 0.98 | 0.84 |

### References

1. Davies, J., G. Shapiro and B. Lowenberg. *Cells and Materials* **3(**3) 1993; pp. 245-56.
2. Gerhart, T., R. Miller, J. Kleshinski and W. Hayes. *J Biomed Mater Res* **22** 1988 ; pp. 1071-82.
3. Kurashina, K., H. Kurita, M. Hirano, J. deBlieck, C. Klein and K. deGroot. *Journal of Materials Science: Materials in Medicine* **6** 1995; pp. 340-7.
4. Tofe, A., G. Brewster and M. Bowerman. *Characterization and Performance of Calcium Phosphate Coatings for Implants* edited by E. Horowitz and J. Parr. Philadelphia: ASTM, pp. 9-15 (1994).
5. Tolman, D. and W. Laney. *Mayo Clin Proc* **68** 1993; pp. 323-31.
6. Levitt, S., P. Crayton, E. Monroe and R. Condrate. *J Biomed Mater Res* **3** 1969; pp. 683-5.
7. Kadiyala, S., N. Jaiswal, S. Bruder. *Tissue Engin* **3(2)** 1997; pp. 173-84.
8. Conklin, J., C. Cotell, T. Bamett and D. Hansen. *Mat Res Soc Symp Proc* **414** 1996; pp. 65-70.
9. Yamashita, K., T. Arashi, K. Kitagaki, S. Yamada and T. Umegaki. *J Am Ceram Soc* **77** 1994; pp. 2401-7.
10. *Biominerals* edited by F. Driessens and R. Verbeeck. Boston: CRC Press (1990).
11. Brown, W., M. Mathew and M. Tung. *Prog Crys Growth Charact* **4** 1981; pp. 59-87.
12. Elliott J. *Structure and Chemistry of the Apatites and Other Calcium Orthophosphates* New York: Elsevier (1994).
13. Meyer, J. and B. Fowler. *Inorg Chem* **21** 1997; pp. 3029-35.
14. Santos, R. and R. Clayton. *American Mineralogist* **80** 1995; pp. 336-44.
15. Brown, P., N. Hocker and S. Hoyle. *J Am Ceram Soc* **74(8)** 1991; pp. 1848-54.
16. Brown, P. and M. Fulmer. *J Am Ceram Soc* **74(5)** 1991; pp. 934-40.
17. Ito, K., Y. Ooi. *CRC Handbook of Bioactive Ceramics* edited by T. Yamamuro, L. Hench and J. Wilson. Boca Raton, Florida: CRC Press, pp. 39-51 (1990).
18. Ohgushi, H., M. Okumura, S. Tamai, E. Shors and A. Caplan. *J Biomed Mater Res* **24** 1990; pp. 1563-70.
19. LeGeros, R., G. Daculsi. *CRC Handbook of Bioactive Ceramics* edited by T. Yamamuro, L. Hench and J. Wilson. Boca Raton: CRC Press, (1990).
20. JCPDS-International Centre for Diffraction Data and American Society for Testing and Materials. *Powder Diffraction File (Inorganic and Organic).* Swarthmore, Pa. JCPDS-International Centre for Diffraction Data. 1991;
21. Clark, N., H. Lunacek and G. Benedek. *Am J Phys* **38(5)** 1970; pp. 575-85.
22. Schumacher, R. *Am J Phys* **54(2)** 1986; pp. 137-41.
23. Bale, C.W., A.D. Pelton, and W.T. Thompson. *FACT Database* [computer program]. Contact: W.T. Thompson, Chemical and Materials Engineering, Royal Military College, Kingston, Canada, K7K 5L0 (1997).
24. Welch, J. and W. Gutt. *J Chem Soc* 1961; pp. 4442-4.
25. Schroeder, L., B. Dickens and W. Brown. *J Solid State Chem* **22** 1977; pp. 253-62.
26. Dickens, B. and W. Brown. *Acta Cryst* **B28** 1972; pp. 3056-65.
27. Nurse, R., J. Welch and W. Gutt. *J Chem Soc* 1959; pp. 1077-83.
28. Elliott, J. *Nature Physical Science* **230** 1971; p. 72
29. Dickens, B., L. Schroeder and W. Brown. *J Solid State Chem* **10** 1974; pp. 232-48.
30. Labarther, J., G. Bonel and G. Montel. *Ann Chim (Paris)* **14th Series 8** 1973; pp. 289-301.
31. Calvo, C. and R. Gopal. *Am Miner* **60** 1975; pp. 120-33.
32. Mathew, M., W. Schroeder, B. Dickens and W. Brown. *Acta Cryst* **B33** 1977; pp.1325-33.
33. Keller, L., P. Rey-Fessler. *Characterization and Performance of Calcium Phosphate Coatings for Implants* edited by E. Horowitz and J. Parr. Philadelphia: ASTM, pp. 54-62 (1994).
34. Shannon, R.D., Acta Cryst. A32., 751, (1976).

## Claims

1. A bioresorbable biomaterial tricalcium phosphate compound comprising calcium, oxygen and phosphorous, wherein a portion of at least one of said elements is substituted with an element having an ionic radius of approximately 0.1 to 0.4Å.

2. A biomaterial compound according to claim 1, wherein a portion of the phosphorous is substituted by at least one element having an ionic radius of approximately 0.1 to 0.4Å.

3. A biomaterial compound according to claim 2, further comprising an additional element having an effective charge to compensate any imbalance of charge resulting from the partial substitution of phosphorous.

4. A biomaterial compound according to claim 1 or 2, wherein said element is silicon.

5. A biomaterial compound according to claim 1 or 4, wherein said compound has a microporous structure.

6. A biomaterial compound according to claim 5, wherein said compound is formed as a macroporous structure comprising an open cell construction with interconnected voids having a pore size of approximately 50 to 1000 microns.

7. A biomaterial compound according to claim 6, wherein said microporous structure is formed by coating said compound onto a reticulated polymer and subsequently removing said polymer through pyrolysis.

8. A biomaterial compound according to claim 5, wherein said compound has a nanoporous structure.

9. A biomaterial compound according to claim 1 or 2, wherein said compound exhibits monoclinic pseudorhombic symmetry and is in the monoclinic space group p2₁a.

10. A biomaterial compound according to claim 1 or 2, wherein said compound is resorbed by the cellular activity of osteoclasts and promotes the generation of new mineralised bone matrix by the activity of osteoblasts.

11. A biomaterial compound according to claim 10, wherein said compound is progressively replaced with natural bone *in vivo.*

12. A biomaterial compound according to claim 10, wherein said compound is essentially insoluble in biological media at human physiological pH 6.4-7.3.

13. A biomaterial compound according to claim 1 or 2, wherein the calcium to phosphorous atomic ratio is in the range of 1.5 to 1.67.

14. A biomaterial compound according to claim 1, wherein said element is boron.

15. A biomaterial composition comprising the compound as claimed in any preceding claim and further comprising a calcium material selected from calcium hydroxyapatite, α-TCP, β-TCP, octacalcium phosphate, tetracalcium phosphate, dicalcium phosphate and calcium oxide.

16. A biomaterial composition comprising the biomaterial compound as claimed in claim 15 and further comprising collagen.

17. A biomaterial compound having the formula: (Ca)ᵢ{(P_{1-x-y-z}BₓC_{y}D_{z})Oⱼ}₂ wherein B, C and D are selected from those elements having an ionic radius of approximately 0.1 to 0.4Å;
X is greater than or equal to zero but less than 1;
Y is greater than or equal to zero but less than 1;
Z is greater than or equal to zero but less than 1; x+y+z is greater than zero but less than 1;
i is greater than or equal to 2 but less than or equal to 4, and
j is equal to 4-δ, wherein δ is greater than or equal to zero but less than or equal to 1.

18. A biomaterial compound according to claim 17, wherein δ is determined by charge compensation of the elements present in the compound.

19. A biomaterial compound according to claim 17 or 18, wherein B is silicon.

20. A biomaterial compound according to claim 17 or 18, wherein B is boron.

21. A biomaterial compound according to claim 17, wherein said compound is selected from the group consisting of Ca₃(P_{0.750}Si_{0.25}O_{3.875})₂ and Ca₃(P_{0.9375}Si_{0.0625}O_{3.96875})₂.

22. A biomaterial composition comprising the biomaterial compound as claimed in any of claims 17 to 21 and further comprising at least one calcium material selected from the group consisting of calcium hydroxyapatite, α-TCP, β-TCP, octacalcium phosphate, tetracalcium phosphate, dicalcium phosphate and calcium oxide.

23. A biomaterial composition according to claim 22, wherein B is silicon and wherein said compound is mixed with calcium hydroxyapatite in a ratio of approximately 20:80 to 80:20.

24. A biomaterial composition according to claim 22, wherein said composition additionally comprises an additive to increase the mechanical toughness and strength of said biomaterial composition.

25. A biomaterial composition according to claim 22, wherein said composition exists as a physical mixture or a solid solution.

26. A composition according to claim 22, wherein the composition exists as a fine or coarse powder, pellets, three-dimensional shaped pieces, macroporous structures and coatings.

27. A composition according to claim 22, wherein said composition is resorbed by the cellular activity of osteoclasts and promotes the generation of new mineralised bone matrix by the activity of osteoblasts.

28. A composition according to claim 22, wherein said composition is used as a carrier and additionally comprises a pharmaceutical agent.

29. A compound according to any one of claims 1-14 or 17-21 or a composition according to any one of claims 15-16 or 22-28 for use as a medicament.

30. The use of a compound according to any one of claims 1-14 or 17-21, or a composition according to any one of claims 15-16 or 22-28 in the manufacture of a medicament for treating bone related clinical conditions.

31. The use of a compound according to any one of claims 1-14 or 17-21, or a composition according to any one of claims 15-16 or 22-28 in the manufacture of a medicament for substituting natural bone at sites of skeletal surgery in human and animal hosts; or for repairing large segmental skeletal gaps and non-union fractures arising from trauma or surgery in human and animal hosts; or for aiding the attachment of implantable prostheses to skeletal sites and for maintaining the long term stability of said prostheses in human and animal hosts; or for providing tissue-engineering scaffolds for bone replacement in human or animal hosts.

## Patentansprüche

1. Bioresorbierbare Biomaterial-Tricalciumphosphat-Verbindung, umfassend Calcium, Sauerstoff und Phosphor, worin ein Teil von mindestens einem der genannten Elemente mit einem Element, das einen Ionenradius von ca. 0,1 bis 0,4 Å aufweist, substituiert ist.

2. Biomaterial-Verbindung nach Anspruch 1, worin ein Teil des Phosphors durch mindestens ein Element, das einen Ionenradius von ca. 0,1 bis 0,4 Å aufweist, substituiert ist.

3. Biomaterial-Verbindung nach Anspruch 2, weiter umfassend ein zusätzliches Element mit einer wirksamen Ladung zur Kompensation eines jedweden Ladungsungleichgewichtes, das aus der teilweisen Substitution von Phosphor resultiert.

4. Biomaterial-Verbindung nach Anspruch 1 oder 2, worin genanntes Element Silicium ist.

5. Biomaterial-Verbindung nach Anspruch 1 oder 4, worin genannte Verbindung eine mikroporöse Struktur aufweist.

6. Biomaterial-Verbindung nach Anspruch 5, worin genannte Verbindung als eine makroporöse Struktur gebildet wird, umfassend einen offenporigen Aufbau mit verbundenen Hohlräumen mit einer Porengröße von ca. 50 bis 1000 Mikron.

7. Biomaterial-Verbindung nach Anspruch 6, worin genannte mikroporöse Struktur durch Beschichten der genannten Verbindung auf ein retikuläres Polymer und anschließendes Entfernen des genannten Polymers durch Pyrolyse gebildet wird.

8. Biomaterial-Verbindung nach Anspruch 5, worin genannte Verbindung eine nanoporöse Struktur aufweist.

9. Biomaterial-Verbindung nach Anspruch 1 oder 2, worin genannte Verbindung eine monokline pseudorhombische Symmetrie zeigt und in der monoklinen Raumgruppe p2₁a vorliegt.

10. Biomaterial-Verbindung nach Anspruch 1 oder 2, worin genannte Verbindung durch die zelluläre Aktivität von Osteoklasten resorbiert wird und die Erzeugung neuer mineralisierter Knochenmatrix durch die Aktivität von Osteoblasten fördert.

11. Biomaterial-Verbindung nach Anspruch 10, worin genannte Verbindung *in vivo* zunehmend gegen natürlichen Knochen ausgetauscht wird.

12. Biomaterial-Verbindung nach Anspruch 10, worin genannte Verbindung in biologischen Medien bei menschlichem, physiologischem pH 6,4-7,3 im Wesentlichen unlöslich ist.

13. Biomaterial-Verbindung nach Anspruch 1 oder 2, worin das Atomverhältnis von Calcium zu Phosphor im Bereich von 1,5 bis 1,67 liegt.

14. Biomaterial-Verbindung nach Anspruch 1, worin genanntes Element Bor ist.

15. Biomaterial-Zusammensetzung, umfassend die Verbindung nach einem vorstehenden Anspruch und weiter umfassend ein Calcium-Material, das aus Calciumhydroxyapatit, α-TCP, β-TCP, Octacalciumphosphat, Tetracalciumphosphat, Dicalciumphosphat und Calciumoxid ausgewählt ist.

16. Biomaterial-Zusammensetzung, umfassend die Biomaterial-Verbindung nach Anspruch 15 und weiter umfassend Kollagen.

17. Biomaterial-Verbindung mit der Formel:
(Ca)ᵢ{(P_{1-x-y-z}BₓC_{y}D_{z})Oⱼ}₂, worin B, C und D aus den Elementen ausgewählt sind, die einen Ionenradius von ca. 0,1 bis 0,4 Å aufweisen;
X größer als oder gleich Null ist, doch kleiner als 1 ist;
Y größer als oder gleich Null ist, doch kleiner als 1 ist;
Z größer als oder gleich Null ist, doch kleiner als 1 ist;
x+y+z größer als Null ist, doch kleiner als 1 ist;
i größer als oder gleich 2 ist, doch kleiner als oder gleich 4 ist, und
j gleich 4-δ ist, worin δ größer als oder gleich Null ist, doch kleiner als oder gleich 1 ist.

18. Biomaterial-Verbindung nach Anspruch 17, worin δ durch den Ladungsausgleich der in der Verbindung gegenwärtigen Elemente bestimmt ist.

19. Biomaterial-Verbindung nach Anspruch 17 oder 18, worin B Silicium ist.

20. Biomaterial-Verbindung nach Anspruch 17 oder 18, worin B Bor ist.

21. Biomaterial-Verbindung nach Anspruch 17, worin genannte Verbindung aus der Gruppe, bestehend aus Ca₃(P_{0,750}Si_{0,25}O_{3,875})₂ und Ca₃(P_{0,9375}Si_{0,0625}O_{3,96875})₂, ausgewählt ist.

22. Biomaterial-Zusammensetzung, umfassend die Biomaterial-Verbindung nach einem der Ansprüche 17 bis 21 und weiter umfassend mindestens ein Calcium-Material, das aus der Gruppe ausgewählt ist, bestehend aus Calciumhydroxyapatit, α-TCP, β-TCP, Octacalciumphosphat, Tetracalciumphosphat, Dicalciumphosphat und Calciumoxid.

23. Biomaterial-Zusammensetzung nach Anspruch 22, worin B Silicium ist und worin genannte Verbindung mit Calciumhydroxyapatit in einem Verhältnis von ca. 20:80 bis 80:20 gemischt ist.

24. Biomaterial-Zusammensetzung nach Anspruch 22, worin genannte Zusammensetzung zusätzlich ein Additiv zur Erhöhung der mechanischen Zähigkeit und Festigkeit der genannten Biomaterial-Zusammensetzung umfasst.

25. Biomaterial-Zusammensetzung nach Anspruch 22, worin genannte Zusammensetzung als eine physikalische Mischung oder eine feste Lösung vorliegt.

26. Zusammensetzung nach Anspruch 22, worin die Zusammensetzung als ein feines oder grobes Pulver, Pellets, dreidimensional geformte Stücke, makroporöse Strukturen und Beschichtungen vorliegt.

27. Zusammensetzung nach Anspruch 22, worin genannte Zusammensetzung durch die zelluläre Aktivität von Osteoklasten resobiert wird und die Erzeugung neuer mineralisierter Knochenmatrix durch die Aktivität von Osteoblasten fördert.

28. Zusammensetzung nach Anspruch 22, worin genannte Zusammensetzung als ein Träger verwendet wird und zusätzlich ein pharmazeutisches Mittel umfasst.

29. Verbindung nach einem der Ansprüche 1-14 oder 17-21 oder eine Zusammensetzung nach einem der Ansprüche 15-16 oder 22-28 zur Verwendung als ein Medikament.

30. Verwendung einer Verbindung nach einem der Ansprüche 1-14 oder 17-21 oder einer Zusammensetzung nach einem der Ansprüche 15-16 oder 22-28 bei der Herstellung eines Medikaments zur Behandlung von knochenspezifischen klinischen Zuständen.

31. Verwendung einer Verbindung nach einem der Ansprüche 1-14 oder 17-21 oder einer Zusammensetzung nach einem der Ansprüche 15-16 oder 22-28 bei der Herstellung eines Medikaments zum Austausch von natürlichem Knochen an Stellen einer Skelettoperation in menschlichen und tierischen Wirten; oder zur Behebung von großen segmentären Skelettlücken und nichtverheilten Frakturen, die durch Verletzung oder Operation in menschlichen und tierischen Wirten entstehen; oder zur Unterstützung der Anbringung von implantierbaren Prothesen an Skelettstellen und zur Aufrechterhaltung der Langzeitstabilität von genannten Prothesen in menschlichen und tierischen Wirten; oder zur Bereitstellung von Gewebe-aufbauenden Gerüsten zum Knochenersatz in menschlichen und tierischen Wirten.

## Revendications

1. Composé phosphate tricalcique de biomatériau biorésorbable comprenant du calcium, de l'oxygène et du phosphore, dans lequel une partie d'au moins un desdits éléments est substituée avec un élément présentant un rayon ionique d'environ 0,1 à 0,4 Å.

2. Composé de biomatériau selon la revendication 1, dans lequel une partie du phosphore est substituée par au moins un élément présentant un rayon ionique d'environ 0,1 à 0,4 Å.

3. Composé de biomatériau selon la revendication 2, comprenant en outre un élément supplémentaire présentant une charge efficace pour compenser tout déséquilibre de charge résultant de la substitution partielle du phosphore.

4. Composé de biomatériau selon la revendication 1 ou la revendication 2, dans lequel ledit élément est du silicium.

5. Composé de biomatériau selon la revendication 1 ou la revendication 4, dans lequel ledit composé présente une structure microporeuse.

6. Composé de biomatériau selon la revendication 5, dans lequel ledit matériau est formé comme une structure macroporeuse comprenant une construction de cellule ouverte avec des vides interconnectés présentant une taille de pore d'environ 50 à 1000 microns.

7. Composé de biomatériau selon la revendication 6, dans lequel ladite structure microporeuse est formée en revêtant ledit composé sur un polymère réticulé et en éliminant ensuite ledit polymère au moyen d'une pyrolyse.

8. Composé de biomatériau selon la revendication 5, dans lequel ledit composé présente une structure nanoporeuse.

9. Composé de biomatériau selon la revendication 1 ou la revendication 2, dans lequel ledit composé exhibe une symétrie pseudorhombique monoclinique et est dans le groupe spatial monoclinique p2₁a.

10. Composé de biomatériau selon la revendication 1 ou la revendication 2, dans lequel ledit composé est résorbé par l'activité cellulaire d'ostéoclastes et stimule la génération d'une nouvelle matrice osseuse minéralisée grâce à l'activité d'ostéoblastes.

11. Composé de biomatériau selon la revendication 10, dans lequel ledit composé est progressivement remplacé par de l'os naturel *in vivo.*

12. Composé de biomatériau selon la revendication 10, dans lequel ledit composé est essentiellement insoluble dans un milieu biologique au pH physiologique humain de 6,4 - 7,3.

13. Composé de biomatériau selon la revendication 1 ou la revendication 2, dans lequel le rapport atomique du calcium sur le phosphore est dans la gamme de 1,5 à 1,67.

14. Composé de biomatériau selon la revendication 1, dans lequel ledit élément est le bore.

15. Composition de biomatériau comprenant le composé tel que revendiqué dans l'une quelconque des revendications précédentes et comprenant en outre un matériau calcique sélectionné parmi l'hydroxyapatite de calcium, α-TCP, β-TCP, le phosphate octacalcique, le phosphate tétracalcique, le phosphate dicalcique et l'oxyde de calcium.

16. Composition de biomatériau comprenant le composé de biomatériau tel que revendiqué à la revendication 15 et comprenant en outre du collagène.

17. Composé de biomatériau présentant la formule (Ca)ᵢ{P_{1-x-y-z}BₓC_{y}D_{z}}Oⱼ}₂, dans laquelle B, C et D sont sélectionnés parmi ces éléments présentant un rayon ionique d'environ 0,1 à 0,4 Å ;
X est supérieur ou égal à zéro mais inférieur à 1 ;
Y est supérieur ou égal à zéro mais inférieur à 1 ;
Z est supérieur ou égal à zéro mais inférieur à 1 ; x+y+z est supérieur à zéro mais inférieur à 1 ;
i est supérieur ou égal à 2 mais inférieur ou égal à 4 ; et
j est égal à 4-δ, dans lequel δ est supérieur ou égal à zéro mais inférieur ou égal à 1.

18. Composé de biomatériau selon la revendication 17, dans lequel δ est déterminé par la compensation de charge des éléments présents dans le composé.

19. Composé de biomatériau selon la revendication 17 ou la revendication 18, dans lequel B est du silicium.

20. Composé de biomatériau selon la revendication 17 ou la revendication 18, dans lequel B est du bore.

21. Composé de biomatériau selon la revendication 17, dans lequel ledit composé est sélectionné parmi le groupe constitué de Ca₃(P_{0,750}Si_{0,25}O_{3,875})₂ et Ca₃ (P_{0,9375}Si_{0,0625}O_{3,96875})₂.

22. Composition de biomatériau comprenant le composé de biomatériau tel que revendiqué dans l'une quelconque des revendications 17 à 21 et comprenant en outre au moins un matériau calcique sélectionné parmi le groupe constitué de l'hydroxyapatite de calcium, α-TCP, β-TCP, le phosphate octocalcique, le phosphate tétracalcique, le phosphate dicalcique et l'oxyde de calcium.

23. Composition de biomatériau selon la revendication 22, dans laquelle B est du silicium et dans laquelle ledit composé est mélangé avec de l'hydroxyapatite de calcium dans un rapport d'environ 20:80 à 80:20.

24. Composition de biomatériau selon la revendication 22, dans laquelle ladite composition comprend en outre un adjuvant pour augmenter la résistance mécanique et la force de ladite composition de biomatériau.

25. Composition de biomatériau selon la revendication 22, dans laquelle ladite composition existe en tant que mélange physique ou solution solide.

26. Composition selon la revendication 22, dans laquelle la composition existe en tant que poudre fine ou grossière, granulés, pièces de forme tridimensionnelle, structures macroporeuses et revêtements.

27. Composition selon la revendication 22, dans laquelle ladite composition est résorbée par l'activité cellulaire d'ostéoclastes et stimule la génération d'une nouvelle matrice osseuse minéralisée grâce à l'activité d'ostéoblastes.

28. Composition selon la revendication 22, dans laquelle ladite composition est utilisée comme support et comprend en outre un agent pharmaceutique.

29. Composé selon l'une quelconque des revendications 1 - 14 ou 17 - 21, ou composition selon l'une quelconque des revendications 15 - 16 ou 22 - 28 à utiliser en tant que médicament.

30. Utilisation d'un composé selon l'une quelconque des revendications 1 - 14 ou 17 - 21, ou composition selon l'une quelconque des revendications 15 - 16 ou 22 - 28 dans la fabrication d'un médicament pour le traitement de conditions cliniques concernant les os.

31. Utilisation d'un composé selon l'une quelconque des revendications 1 - 14 ou 17 - 21, ou composition selon l'une quelconque des revendications 15 - 16 ou 22 - 28 dans la fabrication d'un médicament pour substituer un os naturel au niveau de sites de chirurgie du squelette chez des hôtes humains et animaux ; ou pour réparer des espaces squelettiques segmentaires importants et des fractures non consolidées résultant d'un traumatisme ou d'une chirurgie chez des hôtes humains et animaux ; ou pour aider à fixer des prothèses internes sur des sites du squelette et pour maintenir la stabilité sur le long terme desdites prothèses chez des hôtes humains et animaux ; ou pour fournir des échafaudages de l'ingénierie tissulaire pour la greffe osseuse chez des hôtes humains ou animaux.
